# EUROPEAN PATENT APPLICATION

(11) **EP 2 322 178 A2**
(43) Date of publication of application: **18.05.2011**
(21) Application number: 10191018.0
(22) Date of filing: 12.11.2010
(51) Int. Cl.: A61K 31/4745, A61K 31/7125, A61P 9/10

(54) **Use of toll-like receptor ligands in treating excitotoxic injury, ischemia and/or hypoxia**

(30) Priority: 13.11.2009 US 618691
(71) Applicant: Oregon Health & Science University, Portland, OR 97201 (US)
(72) Inventor: Stenzel-Poore, Mary, M., Lake Oswego, OR 97035 (US); Stevens, Susan, M., Portland, OR 97213 (US)
(74) Representative: Gowshall, Jonathan Vallance

(57) **Abstract**

Methods of protecting cells against cytotoxic insults are provided. The methods involve administering a composition including a Toll-like receptor (TLR) ligand, for instance a TLR7, TLR8, or TLR9 ligand to a subject. The methods are applicable to the protection of neural and non-neural cells. For example, methods of protecting a neural cell against excitotoxic brain injury are provided. Methods for preparing medicaments for the prophylactic treatment of excitotoxic injury, ischemia and/or hypoxia are also provided. Also provided are compositions for use in the described methods.

SEQUENCE LISTING

<110>  Stenzel-Poore, Mary
       Stevens, Susan
       Packard, Amy

<120>  USE OF TOLL-LIKE RECEPTOR LIGANDS IN TREATING EXCITOTOXIC INJURY,
        ISCHEMIA AND/OR HYPOXIA

<130>  899-71282-08

<150>  61/114,934
<151>  2008-11-14

<150>  12/066,260
<151>  2008-11-24

<150>   PCT/US2006/034797
<151>  2006-09-08

<150>  60/715,881
<151>  2005-09-09

<160>  11

<170>  PatentIn version 3.5

<210>  1
<211>  20
<212>  DNA
<213>  Artificial Sequence

<220>
<223>  exemplary oligonucleotide that binds to and activates mouse TLR9

<400>  1
tccatgacgt tcctgacgtt                                                   20


<210>  2
<211>  20
<212>  DNA
<213>  Artificial Sequence

<220>
<223>  exemplary human Class A CpG oligonucleotide

<400>  2
gggggacgat cgtcgggggg                                                   20


<210>  3
<211>  24
<212>  DNA
<213>  Artificial Sequence

<220>
<223>  exemplary human Class B CpG oligonucleotide

<400>  3
tcgtcgtttt gtcgttttgt cgtt                                              24


<210>  4
<211>  25
<212>  DNA
<213>  Artificial Sequence

<220>

<223>  exemplary human Class C CpG oligonucleotide

<400>  4
tcgtcgtcgt tcgaacgacg ttgat                                          25


<210>  5
<211>  22
<212>  DNA
<213>  Artificial Sequence

<220>
<223>  exemplary human Class B CpG oligonucleotide

<400>  5
tgactgtgaa cgttcgagat ga                                             22


<210>  6
<211>  20
<212>  DNA
<213>  Artificial Sequence

<220>
<223>  CpG oligonucleotide present in Kmix

<400>  6
atcgactctc gagcgttctc                                                20


<210>  7
<211>  12
<212>  DNA
<213>  Artificial Sequence

<220>
<223>  CpG oligonucleotide present in Kmix

<400>  7
tcgagcgttc tc                                                        12


<210>  8
<211>  12
<212>  DNA
<213>  Artificial Sequence

<220>
<223>  CpG oligonucleotide present in Kmix

<400>  8
tcgttcgttc tc                                                        12


<210>  9
<211>  20
<212>  DNA
<213>  Artificial Sequence

<220>
<223>  CpG oligonucleotide present in Dmix

<400>  9
ggtgcatcga tgcagggggg                                                20


<210>  10
<211>  20
<212>  DNA

<213>  Artificial Sequence

<220>
<223>  CpG oligonucleotide present in Dmix

<400>  10
ggtgcaccgg tgcagggggg                                                   20


<210>  11
<211>  20
<212>  DNA
<213>  Artificial Sequence

<220>
<223>  CpG oligonucleotide present in Dmix

<400>  11
ggtgcatcga tgcagggggg                                                   20

## Description

### CROSS REFERENCE TO RELATED APPLICATIONS

This application claims the benefit of the filing date of U.S. Provisional Application No. 61/114,934 filed November 14, 2008 and is a continuation-in-part of US Application No. 12/066,260 filed November 24, 2008, which is the U.S. § 371 National Stage of International Application No. PCT/US2006/034797, filed September 8, 2006, which in turn claims benefit of the filing date of U.S. Provisional Application No. 60/715,881 filed September 9, 2005.

### ACKNOWLEDGMENT OF GOVERNMENT SUPPORT

Aspects of this invention were made with United States government support pursuant to grant no. NS 35965 and NS 050567 from the National Institute of Neurological Disorders and Stroke (NINDS). The United States government has certain rights in the invention.

### FIELD

The present disclosure relates to the treatment and prevention of cellular and organ damage due to excitotoxic injury, ischemia and/or hypoxia by administering an agent that binds to and activates a cellular Toll-like receptor (TLR).

### BACKGROUND

The need for therapies to reduce the adverse effects of ischemic and other hypoxic conditions is enormous. For example, transient ischemic attacks (TIA's) precede infarction in 25-50% of patients with occlusive cerebral vascular disease, and 50% of patients that undergo coronary artery bypass surgery (CABG) suffer permanent cognitive decline from intraoperative emboli. Perioperative treatment of CABG patients alone (336,000 annually) could reduce stroke incidence and morbidity significantly. Furthermore, individuals who have had a stroke are at high risk of recurrent stroke (25-40% within 5 years).

Decades of research investigating stroke pathogenesis and treatment have revealed robust neuroprotective treatments in the laboratory, however, all have failed to translate into treatments for patients (Plum, J. Am. Med. Assoc., 285:1760-1761, 2001; DeGraba and Pettigrew, Neurol. Clin. 18:475-493, 2000). The failure of a pharmacologic approach to induce neuroprotection in humans may be due to trial design, dose response or time window issues of selected compounds or side effects of study agents. However, all cytoprotective trials over a 25 year period have been negative.

Ischemic tolerance in the brain—in which one or more brief ischemic insults increase resistance to subsequent injurious ischemia—is a powerful adaptive defense that involves an endogenous program of neuroprotection (Nandagopal et al., J. Pharm. Exp. Ther. 297:474-478, 2001; Chen et al., J. Cereb. Blood Flow Metab. 16:566-577, 1996; and reviewed in Dirnagl et al., Trends Neruosci. 26:248-254, 2003). This neuroprotective program sets into motion a complex cascade of signaling events, leading to synthesis of new proteins, which ultimately re-programs the cellular response to subsequent injury. The sequence of events that leads to ischemic tolerance is only partially known (Nandagopal et al., J. Harm. Exp. Ther. 297:474-478, 2001), although evidence is emerging that diverse stimuli that trigger preconditioning may share a common pathway that confers neuroprotection (Gonzalez-Zulueta et al., Proc. Natl. Acad. Sci. USA 97:436-441, 2000; Kasischke et al., Neurosci. Lett. 214:175-178, 1996; Gidday et al., J. Cereb. Blood Flow Metab. 19:331-340, 1999; Kato et al., Neurosci. Lett. 139:118-121, 1992*).*

Tolerance to ischemic brain injury can be induced by several distinct preconditioning stimuli including non-injurious ischemia, cortical spreading depression, brief episodes of seizure, exposure to anesthetic inhalants, and low doses of endotoxin (lipopolysaccharide, LPS) *(*Simon et al., Neurosci. Lett. 163:135-137, 1993; Chen and Simon, Neurology, 48:306-311, 1997; Kitagawa et al., Brain Res. 528:21-24, 1990; Kobayashi et al., J. Cereb. Blood Flow Metab. 15:721-727, 1995; Kapinya et al., Stroke 333:1889-1898,2002; Towfighi et al., Dev. Brain. Res. 113:83-95, 1999). Although the mechanisms that underlie these processes, and preconditioning in general, are not well understood, they may share a common link that small doses of an otherwise harmful stimulus induce protection against subsequent injurious challenge (Dirnagl et al., Trends Neruosci. 26:248-254, 2003).

**LPS**, given in small doses, confers profound neuroprotection against subsequent stroke. However, LPS is poorly tolerated by human and animal subjects. Therefore, alternatives to LPS for preconditioning against stroke and treatment of stroke and other hypoxic and/or excitotoxic injuries are needed.

### SUMMARY

The present disclosure relates to methods and compositions for (1) treating and (2) protecting cells against cytotoxic insult. The methods disclosed herein are applicable to the treatment and protection of neural as well as non-neural cells, and are relevant for the treatment and prevention of adverse outcomes due to diverse medical conditions, including epilepsy, traumatic brain injury, *in utero* hypoxia, ischemic events (including stroke) and Alzheimer's disease, as well as surgical and non-surgical trauma.

In particular examples, the methods include systemically administering a Toll-like receptor (TLR) ligand or other agent that can activate a TLR (such as a TLR antibody agonist) to a subject in order to treat a cell in a subject following an excitotoxic injury, ischemia, hypoxia, or combinations thereof. The TLR ligand or other agonist thereby treats the cell for excitotoxic injury, ischemia, hypoxia, or combinations thereof. In particular examples, the TLR ligand or other agonist, such as a TLR3 ligand (for example poly I:C or analog thereof) or a TLR7 ligand (for example gardiquimod), is administered after the subject suffers from excitotoxic injury, ischemia, or hypoxia. Thus, the disclosure relates to methods for the subsequent treatment of cellular injury and death due to cytotoxic insults, such as excitotoxic, ischemic and/or hypoxic events.

In other examples, the methods involve systemically administering a TLR ligand or other agonist (such as a ligand or agonist for any of TLRs 1-10) that elicits a preconditioning effect to a subject. The TLR ligand/agonist is administered to protect a cell in a subject from excitotoxic injury, or injury resulting from ischemia, hypoxia, or combinations thereof. Typically, the TLR ligand/agonist is administered prior to the excitotoxic, ischemic, and/or hypoxic event, or prior to one or more events in a series of events or during the occurrence of an ongoing or progressive process. Thus, the disclosure relates to methods for the prophylactic treatment of cellular injury and death due to cytotoxic insults, such as excitotoxic, ischemic and/or hypoxic events.

The foregoing and other objects and features of the disclosure will become more apparent from the following detailed description, which proceeds with reference to the accompanying figures.

### BRIEF DESCRIPTION OF THE DRAWINGS

**FIG. 1** is a bar graph illustrating neuroprotection following *in vitro* treatment with an exemplary CpG oligonucleotide. Values are group means +/- SEM; *P<0.05.

**FIG. 2** is a bar graph illustrating neuroprotection following *in vitro* treatment with imiquimod. Values are means +/- SEM.

**FIG. 3** is a bar graph illustrating NF-_{K}B induction following *in vitro* treatment with an exemplary CpG oligonucleotide. 293-hTLR9 cells transfected with an NFκB inducible reporter plasmid (pNifty2-SEAP) were treated with CpG (5µM). NFκB induction of alkaline phosphatase expression is indicated as hydrolysis of pNpp at 405nm.

**FIG. 4** is a bar graph illustrating neuroprotection *in vivo* following treatment of mice with an exemplary CpG oligonucleotide. CpG treatment was given 72 hours prior to induced stroke. Values are group means +/- SEM; * p<0.05, **p<0.001.

**FIG**. 5 is a bar graph illustrating a time course of preconditioning in vivo with an exemplary CpG oligonucleotide. CpG dose 20ug/mouse (0.8mg/kg). Values are group means +/- SEM; **p<0.0001.

**FIG. 6** is a bar graph illustrating neuroprotection *in vivo* following treatment of mice with imiquimod. Values are group means ± SEM; (saline n=6, imiquimod n=3); * p<0.05.

**FIG. 7** is a bar graph illustrating neuroprotection following *in vitro* treatment with Gardiquimod (GDQ). Gardiquimod, a TLR7 agonist, protected primary cortical enriched neuronal cultures from OGD-induced cell death. Mouse cortical neuronal cultures were treated with 0.1 or 1 µg/ml GDQ 24 hours prior to oxygen-glucose deprivation-treatment (OGD; 3 hours). Cell death was determined by propidium iodide staining 24 hours following OGD. Data are mean % cell death ± SEM. *p<0.05, ^{**}p<0.01 versus media-treated OGD controls. N = 3 ― 5 experiments.

**FIG. 8** is a bar graph illustrating neuroprotection *in vivo* following treatment of mice with Gardiquimod. Preconditioning with Gardiquimod dose-dependently induced neuroprotection *in vivo.* Mice received various doses of GDQ (subcutaneous; 0.2 - 1.6 mg/kg) 72 hours prior to ischemic challenge (60 minutes middle cerebral artery occlusion (MCAO)). The % of hemisphere with infarction was determined 24 hours later using triphenyltetrazolium chloride (TTC) staining. *p<0.05, **p<0.01 versus saline-injected controls. Values are group means ± SEM. N = 5 - 8.

**FIGS. 9A and 9B** are bar graphs illustrating neuroprotection following *in vivo* treatment with Gardiquimod. A preconditioning dose of Gardiquimod improved neurological deficit. Mice received various doses of GDQ (subcutaneous; 0.2 - 1.6 mg/kg) 72 hours prior to ischemic challenge (60 minutes MCAO). All 3 doses of GDQ reduced neurological deficits (both general and focal) 24 hours post MCAO. **p<0.01 1 versus saline-treated controls. Data presented as group means ± SEM. N = 5-8.

**FIGS. 10A and 10B** are bar graphs illustrating neuroprotection following *in vitro* treatment with the TLR7/8 ligand CL075. Values are means +/- SEM.

**FIG. 11** is a bar graph illustrating neuroprotection following *in vitro* treatment with polyinosinic polycytidylic acid (Poly I:C), a TRL3 ligand. Mixed cortical cultures were treated 24 hours prior to OGD with increasing doses of Poly I:C. Cell death was assessed by PI staining performed 24 hours after. Mean +/- SEM are shown; ***p<0.00, *p<0.05 versus media treated OGD control. N=2-4 individually repeated experiments. Poly I:C treatment at the highest dose without exposure to OGD did not induce cell death.

**FIGS. 12A and 12B** is a bar graph and digital image, respectively, illustrating that administration of Poly I:C prior to ischemia reduces damage in *an in vivo* model of stroke. Mice were given an intraperitoneal injection of poly I:C (25 µg) three days prior to middle cerebral artery occlusion surgery (45 minutes). 72 hours following surgery the brains were stained with TTC and infarct volume was assessed. Mean +/- SEM are shown. *p<0.05 versus saline treated controls.

**FIG. 13** is a bar graph illustrating that administration of poly I:C prior to ischemia reduces damage in *an in vivo* model of renal ischemia. Mice were given an intraperitoneal injection of poly I:C (25 µg) 48 hours prior to bilateral renal clamping (45 minutes). 48 hours following reperfusion serum creatinine levels were assessed. Mean +/- SEM are shown. *p<0.001 versus baseline.

**FIG. 14** is a bar graph illustrating that administration of poly I: C following ischemia reduces damage in *an in vitro* model of stroke. Mean +/- SEM are shown. ***p<0.001 versus media treated OGD control. N = 2-4 individually repeated experiments.

**FIG. 15** is a bar graph showing that TLR3 is a protective pathway in the context of ischemia. Mean +/- SEM are shown. *p<0.05 versus wild type controls.

**FIGS. 16A and 16B** are bar graphs showing that poly I:C increases (A) TNF-α and (B) IFN-ß levels. Mean +/- SEM are shown. ***p<0.001 versus saline.

**FIG. 17** is a bar graph illustrating that administration of resiquimod prior to ischemia reduces damage in *an in vivo* model of cerebral ischemia. Mice were given an s.c. injection of resiquimod (RSQ, 0.8 mg/kg) 72 hours prior to MCAO. 24 hours later infarct size was assessed. Mean +/- SEM are shown. _{**}p<0.01 versus baseline.

**FIG. 18** is a bar graph illustrating that IRF3 is a mediator of LPS preconditioning. Data shown are group means ±SEM; * p<0.05 by Two way ANOVA with Bonferroni post test, n = 7-10 per group.

**FIGS. 19A and B** are bar graphs illustrating neuroprotection *in vivo* following treatment of rhesus macaques with an exemplary CpG oligonucleotide mixture K-mixgiven 3 days prior to induced stroke (60 min, ACA/MCAO). A) Quantification of infarct sizes on T2 images showed that reduction of infarction by 0.06mg/kg of CpG approached significance (p<0.055), and the higher dose (0.3mg/kg) reduced infarct damage by ∼2-fold (*p=0.0045). B) CpG-treated animals showed greater (1.4-fold) motor function than vehicle treated animals at 2 days following stroke based on a modified Spetzler evaluation.

**FIG. 20** is a schematic drawing showing cellular effects of TLR3 and TLR4 signaling.

### SEQUENCE LISTING

The nucleic and amino acid sequences listed herein and/or in the accompanying sequence listing are shown using standard letter abbreviations for nucleotide bases as defined in 37 C.F.R. 1.822. Only one strand of each nucleic acid sequence is shown, but the complementary strand is understood as included by any reference to the displayed strand.

SEQ ID NO:1 (5'-tccatgacgttcctgacgtt-3') is an exemplary oligonucleotide that binds to and activates mouse TLR9.

SEQ ID NO:2 (5'-gggggacgatcgtcgggggg-3') is an exemplary human Class A CpG oligonucleotide.

SEQ ID NO:3 (5'-tcgtcgttttgtcgttttgtcgtt-3') is an exemplary human Class B CpG oligonucleotide.

SEQ ID NO:4 (5'-tcgtcgtcgttcgaacgacgttgat-3') is an exemplary human Class C CpG oligonucleotide.

SEQ ID NO:5 (5'-tgactgtgaacgttcgagatga-3') is an exemplary human Class B CpG oligonucleotide.

SEQ ID NO: 6-8 are exemplary human CpG oligonucleotides that make up a composition called Kmix.

SEQ ID NO: 9-11 are exemplary human CpG oligonucleotides that make up a composition called Dmix.

### DETAILED DESCRIPTION

The present disclosure concerns methods for treating or protecting *cells in vivo,* in the context of a living multicellular organism, from the adverse effects of cytotoxic insults, such as excitotoxic, ischemic, or hypoxic events (or combinations thereof). More specifically, methods disclosed herein involve either preconditioning cells to increase tolerance to subsequent excitotoxic, ischemic and/or hypoxic events, or treating cells following previous excitotoxic, ischemic and/or hypoxic events. The present disclosure provides novel methods, based on the observation that TLR ligands are cytoprotective when used in a preconditioning regimen, for protecting cells against excitotoxic injury, ischemia and hypoxia. In addition, some TLR ligands (such as a TLR3, for example poly I:C) can treat the adverse effects of previous excitotoxic, ischemic and/or hypoxic events.

In one example, administration of TLR ligands or other TLR agonists (e.g., prior to an excitotoxic, ischemic or hypoxic event) induces cellular and metabolic changes by modifying the genomic response program, which results in resistance to subsequent damage that would otherwise result from excessive electrochemical activity and/or oxygen deprivation. In another example, administration of TLR ligands/agonists (e.g., following an excitotoxic, ischemic or hypoxic event) induces cellular and metabolic changes by modifying the genomic response program, which results in treatment of damage that can results from excessive electrochemical activity and/or oxygen deprivation.

Thus, one aspect of the disclosure concerns methods of treating a cell (or population of cells, or a tissue, organ or organism) that has received a previous cytotoxic insult, including excitotoxic injury, ischemia, hypoxia or a combination of thereof, with a TLR ligand or other agonist (such as a TLR antibody agonist). In one example, the TLR ligand is not LPS (for example not a TLR4 ligand). In a specific example, the ligand or agonist is one that can induce high levels of interferon-α and -ß (IFN-α and-ß) and interferon regulated genes, such as TLR3 and TLR7 ligands/agonists. The methods disclosed herein are applicable to different cell types that can suffer from excitotoxic, ischemic and/or hypoxic injury, which are amenable to treatment. For example, neural cells (including, e.g., hippocampal neurons and cortical neurons), muscle cells (including cardiac, smooth and striated muscle cells), hepatic cells, renal cells, endothelial cells, and certain cells of the immune system (e.g., dendritic cells, macrophages, lymphocytes, and microglia) can be treated for excitotoxic injury, ischemia and/or hypoxia using the methods disclosed herein. In one example, this aspect of the disclosure includes treating a cell in a subject suffering from excitotoxic injury or injury due to ischemia or hypoxia (or combinations thereof) by systemically administering to the subject a composition that includes a TLR ligand, thereby treating the cell having an excitotoxic injury, or injury resulting from ischemia, hypoxia, or combinations thereof.

For example, the method can include treating a neural cell in a subject having an excitotoxic injury by systemically administering to the subject a ligand that binds to and activates a TLR expressed by at least one cell of the central nervous system or the periphery (such as administering the TLR ligand after the excitotoxic event), thereby treating the neural cell in the subject having the excitotoxic injury. Administering the TLR ligand can result in increased production of a neuroprotective cytokine. In some examples, a subject who has suffered a previous excitotoxic event is selected prior to administration of the TLR ligand.

In another example, the method can include treating a non-neural cell (such as a renal cell) in a subject having suffered ischemia by systemically administering to the subject a ligand that binds to a TLR expressed by at least one cell of a tissue other than the central nervous system (such as administering the TLR ligand after the ischemic event), thereby treating the non-neural cell. Administering the TLR ligand can result in increased production of a cytoprotective cytokine. In some examples, a subject who has suffered a previous ischemic event is selected prior to administration of the TLR ligand.

In another aspect, the disclosure concerns methods of protecting a cell (or population of cells, or a tissue, organ or organism) against cytotoxic insult, including excitotoxic injury, ischemia, hypoxia or a combination of thereof. The methods disclosed herein are applicable to different cell types susceptible to excitotoxic, ischemic and/or hypoxic injury, which are amenable to preconditioning. For example, neural cells (including, e.g., hippocampal neurons and cortical neurons), muscle cells (including cardiac, smooth and striated muscle cells), hepatic cells, renal cells, lymphocytes, and endothelial cells can be protected against excitotoxic injury, ischemia and/or hypoxia using the methods disclosed herein. Additionally, certain cells of the immune system, including dendritic cells, macrophages and microglia are amenable to preconditioning. In one example, this aspect of the disclosure includes protecting a cell in a subject from excitotoxic injury or injury due to ischemia or hypoxia (or combinations thereof) by systemically administering to the subject a composition that includes a TLR ligand, thereby protecting the cell against excitotoxic injury, ischemia, hypoxia, or combinations thereof. In some examples, a subject who is at risk for an excitotoxic injury, ischemia, hypoxia, or combinations thereof is selected prior to administration of the TLR ligand.

The disclosed methods can be utilized to (1) treat cells previously subjected to or (2) protect cells against cytotoxic insult, for example, arising from excitotoxic, ischemic and/or hypoxic events. That is, the methods are useful for treating or protecting cells for a broad range of events and occurrences that include an excitotoxic, ischemic or hypoxic component, or a combination thereof. Excitotoxic injury results from excessive stimulation of cells (typically neural cells in the CNS) by certain neurotransmitter (e.g., glutamate) receptors. For example, excitotoxic injury can be a result of a condition that causes excessive chemical or electrical activity in the brain or it can be a result of conditions that cause a decrease in inhibitory or regulatory functions of the brain. Excitotoxic injury in the brain is associated with a variety of conditions with disparate etiologies and symptoms, including epilepsy, traumatic brain injury and Alzheimer's disease. Hypoxia in the central nervous system (CNS) can be associated with ischemic events (such as cerebrovascular ischemia, stroke, myocardial ischemia due to narrowing or blockage of the vessels of the heart, iatrogenic ischemia, due to surgical procedures, and the like). In addition, hypoxia can occur *in utero* due to conditions such as inadequate placental function (for example, due to abrupio placentae), preeclamptic toxicity, prolapse of the umbilical cord, or complications from anesthetic administration. Ischemic events outside the CNS can also result in injury to tissues and organs, including kidney, liver, lung, intestine, and muscle. Such injury can be the result of vascular disease or injury, as well as a complication of surgical procedures (e.g., cardiovascular surgery). Additionally, injury by some hypoxic events (such as strokes) involves an excitotoxic component as well as a hypoxic component and are, in some but not all cases related to ischemic events. In one example, the subject treated has suffered from or is at risk for: atrial fibrillation, one or more transient ischemic events, a stroke, hypertension, or combinations thereof. Thus, it will be appreciated that these terms can be extensively overlapping, but are not necessarily coextensive in every condition that is amenable to treatment or preconditioning. For simplicity of reference, in the context of this disclosure, the term "cytotoxic insult" is used to refer to any of these conditions, separately or in any combination. The methods disclosed herein are useful for preventing or treating cellular damage in any (and/or all) of these conditions.

Accordingly, the methods can involve selecting a subject (1) who has previously had or (2) is at risk for, one or more of an excitotoxic, ischemic or hypoxic event. In the context of determining whether a subject has previously had one or more of excitotoxic, ischemic or hypoxic event, such events are indicated by a variety of medical as well as non-medical indicators, as would be recognized by one of ordinary skill in the art. For example characteristic symptoms of stroke are sudden weakness, numbness, or paralysis (usually unilateral and in the arm, leg, or face), also a sudden and severe headache, full or partial loss of vision, dizziness and loss of balance, loss of memory, loss of consciousness, and difficulty speaking or understanding language may be observed. Biological signs of ischemic events may be observed using magnetic resonance imaging (MRI) or other techniques.

In the context of the preconditioning methods described herein, risk is indicated by a variety of medical as well as non-medical indicators, as would be recognized by one of ordinary skill in the art. For example, various cardiovascular signs and symptoms, such as atrial fibrillation, angina pectoris, hypertension, transient ischemic attacks and prior stroke, are all indicators of risk that can be used to select a subject for administration of preconditioning agent according to the methods disclosed herein. Similarly, surgical procedures, especially those specifically involving the cardiovascular system, such as endarterectomy, pulmonary bypass and coronary artery bypass surgeries, or abdominal surgeries are indicators of risk that can be used to select a subject for administration of a TLR ligand. Intestinal ischemic events including chronic or acute mesenteric ischemia are associated with risk factors including advanced age, patients with postprandial abdominal pain and weight loss, chronic or acute colitis, cardiac arrhythmia, recent heart attack or prior emboli, prior arterial insufficiency, diabetes, hypertension, trauma, congestive heart failure, infection or inflammation and prior use of vasoconstrictor drugs.

In addition, non-medical indicators of risk, for example, pertaining to behaviors or activities that are statistically associated with an increased likelihood of injuries, can include an excitotoxic or hypoxic component. For example, traumatic brain injury (regardless of its cause) frequently involves an excitotoxic (and can also include a hypoxic) component. Thus, participation in activities that increase the risk of traumatic brain injury are indicators that can be used to select a subject for administration of a TLR ligand. Such activities include, for example, motorcycle riding, motor vehicle racing, skiing, contact sports (such as, football, hockey, rugby, soccer, lacrosse, martial arts, boxing and wrestling), and the like. Additionally, impacts or wounds resulting from gunshot or explosives frequently cause traumatic brain injury. Accordingly, activities that are associated with an increased risk of gunshot wounds or injury caused by explosive devices (for example, in combat or military situations) are indicators of risk that can be used to select a subject for treatment according to the methods disclosed herein.

Typically, a TLR ligand-containing composition is administered to a subject (such as a human subject) having previously had or at risk for a cytotoxic insult, such as an excitotoxic, ischemic and/or hypoxic event. Commonly, the composition containing the TLR ligand is a pharmaceutical composition or medicament, formulated for administration to a subject. Such compositions commonly include a pharmaceutical carrier or excipient. Generally, the composition is formulated based on the intended route of administration. Suitable routes of administration include intranasal, oral, transdermal, subcutaneous, intrathecal, intravenous, intramuscular, and intraperitoneal routes, and appropriate pharmaceutical carriers for these administration routes are well known in the art. Thus, the use of a TLR ligand in the preparation of a medicament for the treatment or prevention of an excitotoxic injury, ischemia or hypoxia is a feature of this disclosure.

In one example, the TLR ligand is administered after an event or activity associated with (e.g., that increases the risk of) excitotoxic injury, ischemia and/or hypoxia, in order to treat the adverse effects of such an event. In some examples, the TLR ligand or agonist is administered with other therapeutic agents, such as tissue plasminogen activator (tPA). In one example, the TLR ligand is administered in at least one dose at least 1 hour after to the excitotoxic, ischemic or hypoxic event, such as at least 2, 4, 6, 12, 24, 48 or 72 hours after the excitotoxic, ischemic or hypoxic event, for example at least 7 days, at least 10 days, or at least 30 days after the excitotoxic, ischemic or hypoxic event. Optionally, multiple doses of the TLR ligand-containing composition are administered after the excitotoxic, ischemic or hypoxic event. For example, two, or three, or more doses can be administered on separate occasions following the event. In such cases, a first dose is typically given between 1 and 72 hours, at seven days, at six days, at five days, at four days, at three days, at two days, or at 1 day following the event. One or more subsequent administrations of the compositions can be made at any subsequent time point, such as at seven days, at six days, at five days, at four days, at three days, at two days, at 24 hours or at 12 hours following the event.

In another example, the TLR ligand is administered prior to an event or activity associated with (e.g., that increases the risk of) excitotoxic injury, ischemia and/or hypoxia, in order to protect the cells from adverse consequences resulting from such events. For example, at least one dose of the TLR ligand-containing composition can be administered at least 10 hours prior to the event or activity, in order to better realize the preconditioning effect of administration. Usually, the composition is administered at least 24 hours before the event or activity. The protective effects of a single administration of a TLR ligand can last for greater than one week (e.g., up to about 10 days, or more). Thus, in the case of an isolated event, that is, an event that is not predicted to be a recurring event, such as a surgical operation, the composition is given prior to the commencement of the event, such as about 10 hours, or about 12 hours, or about 24 hours prior to the event or activity, and can be given up to about 1 week prior to the event. Optionally, multiple doses of the composition are administered prior to the commencement of the event (e.g., surgery). For example, two, or three, or more doses can be administered on separate occasions preceding the event. In such cases, a first dose is typically given between 8 and 10 days, at seven days, at six days, at five days, at four days, at three days, at two days, or at 1 day prior to the event. One or more subsequent administrations of the compositions can be made at any subsequent time point, such as at seven days, at six days, at five days, at four days, at three days, at two days, at 24 hours or at 12 hours prior to the event.

In the case of a recurrent event, such as repeated engagement in a contact sport, multiple administrations are given, the ultimate dose (that is, the most recent dose prior to the event) being given prior (such as, at least 10 hours, or up to about 1 week, prior) to the event or activity. Similarly, in the case of an ongoing event, such as in the case of Alzheimer's disease, multiple administrations are given, for example on a predetermined schedule, such as at weekly intervals. The individual treatment regimen can be customized to the particular subject event or activity, such that the treatment or protective effects of the TLR ligand are optimized under the particular circumstances for the particular subject.

Typically, the dose of the composition including the TLR ligand administered is a treatment or a preconditioning dose. That is, a dose of the composition is administered that is sufficient to induce cellular changes (for example, in the genomic response) that (1) treat a cell having an injury resulting from a previous cytotoxic insult, such as an excitotoxic, ischemic or hypoxic event or (2) protect the cell against injury resulting from a subsequent cytotoxic insult, such as an excitotoxic, ischemic or hypoxic event. Methods for detecting such genomic changes are described herein, e.g., in Example 2. Exemplary doses that can be used in the methods provided herein (for example, in a human) includes at least 0.005 mg/kg, such as at least 0.05 mg/kg, at least 0.01 mg/kg, at least 0.1 mg/kg, at least 1 mg/kg, or at least 2 mg/kg of the TLR ligand (such as a CpG oligonucleotide, poly I:C). In one example, the dose contains no more than about 0.2 mg/kg, no more than about 0.5 mg/kg, no more than about 1 mg/kg, or no more than about 2 mg/kg of the TLR ligand. For example, a dose can include between 0.01 mg/kg and 1 mg/kg, 0.005 mg/kg to 5 mg/kg, 0.02 mg/kg to 0.9 mg/kg of a TLR ligand, such as between 0.05 mg/kg and 5 mg/kg. Certain exemplary doses include about 0.07, about 0.08, about 0.09, about 0.10, about 0.12, about 0.15, about 0.9, about 1, or about 2 mg/kg of a TLR ligand. Precise doses of the composition employed will depend upon the stimulatory capacity of the individual ligand formulation and human *in vivo* absorption, distribution, metabolism and ultimately the bioavailability of the composition.

Following administration of a composition containing one or more TLR ligands or other agonist, the ligand/agonist binds to and activates an appropriate TLR (such as any of TLRs1-9). For instance, binding of TLR9 by a suitable CpG oligonucleotide ligand results in the activation of intracellular signaling pathways that modify the genetic program in cells expressing TLR9. Similarly, binding of TLR3 by a poly I:C ligand results in the activation of intracellular signaling pathways that modify the genetic program in cells expressing TLR3. These modifications in the genomic response include an increase in the production of certain cytoprotective cytokines. For example, binding of a CpG oligonucleotide to TLR9 on the cell surface of certain immune cells, such as B cells, dendritic cells, macrophages and microglial cells induces production of transforming growth factor-beta (TGFβ), tumor necrosis factor-alpha (TNFα) and type I interferons, such as interferon-beta (IFNß). Similarly, binding of poly I:C to TLR3 on the cell surface of certain immune cells, such as B cells, dendritic cells, and macrophages induces production of TNFα. Thus, representative methods disclosed herein involve administering a TLR ligand (such as a poly I:C or a CpG oligonucleotide) capable of inducing production of one or more cytoprotective cytokines, such as TGFß, TNFα, and IFNß. In some examples, the cytoprotective cytokine is produced by a non-neural cell, such as a B cell, a dendritic cell, a macrophage or a microglial cell.

TLR ligands and other agonists that can be used in the disclosed methods are known in the art (see Table 1 above and Examples below). Binding of these ligands/agonists to their appropriate receptor can be used to induce cellular signaling pathways in the methods provided herein. In one example, the TLR ligand is a TLR1, TLR2, TLR3, TLR4, TLR5, TLR6, TLR7, TLR8, or TLR9 ligand. In some examples the TLR1 ligand is Pam(3)Cys-Ser-(Lys)(4), the TLR2 ligand is MALP-2, the TLR3 ligand is poly I:C or structurally related analogs such as poly(I:C₁₂U) (Ampligen®) or dsRNA, the TLR4 ligand is LPS, the TLR5 ligand is flagellin, the TLR6 ligand is a diacyl lipopeptide, the TLR7 ligand is gardiquimod or imiquimod, the TLR7/8 ligand is resiquimod, and the TLR9 ligand is a CpG oligonucleotide (such as any of SEQ ID NOS: 1-5) or a mixture of CpG oligonucleotides, such as Kmix or Dmix (see below).

| **Exemplary Human CpG ODN mixes** | |
|---|---|
| **Kmix (SEQ ID NO:)** | **Dmix (SEQ 1D NO:)** |
| **K3:** ATCGACTCTCGAGCGTTCTC (6) | **D19:** GGtgcatcgatgcaggGGGG (9) |
| **K23:** TCGAGCGTTCTC (7) | **D29:** GGtgcaccggtgcaggGGGG (10) |
| **K123:** TCGTTCGTTCTC (8) | **D35:** GGtgcatcgatgcaggggGG (11) |
| **Nucleotides shown in CAPS are phosphorothioate modified.** | |

In one example, the TLR ligand is a TLR3 ligand, such as poly I:C or structurally related analogs thereof, such as poly(I:C₁₂U) (Ampligen@). TLR3 ligands can be used in compositions for, or in the preparation of a medicament for, the treatment of a previous excitotoxic injury, ischemic event or hypoxic event. As the TLR3 ligand poly I:C signals through both TLR3 as well as the melanoma differentiation-associated gene-5 (MDA5), one skilled in the art will appreciate that ligands and agonists of MDAS can be used alternatively or in addition to TLR ligands/agonists in the disclosed methods.

In one example, the TLR ligand is a TLR9 ligand, such as a CpG oligonucleotide. Numerous CpG oligonucleotides have been described, and are known to bind to TLR9 and induce cellular signaling pathways. Any of these oligonucleotides can be used in the context of composition for treating a cell having previously been subjected to excitotoxic, ischemic, or hypoxic injury or preconditioning a cell against excitotoxic, ischemic, or hypoxic injury. To increase *in vivo* stability of the CpG oligonucleotide, the oligonucleotide can be modified by the inclusion of one or more phosphorothioate modified nucleotides. Exemplary oligonucleotide sequences suitable for use in mouse (SEQ ID NO: 1) and human (SEQ ID NOS: 2-11) are provided. One skilled in the art will appreciate that combinations of such oligonucleotides can be used, such as the Kmix and Dmix compositions described above.

Another aspect of the disclosure relates to methods of (1) protecting neural cells (including hippocampal and cortical neurons) against excitotoxic brain injury and (2) treating neural cells (including hippocampal and cortical neurons) following an excitotoxic brain injury. Such methods involve systemically administering to a subject an agent that binds to a TLR expressed on a cell of the periphery or in the central nervous system (CNS). The peripheral or CNS cells that express TLRs can be non-neural cells. For example, the non-neural cells can be immune cells, such as T or B cells, dendritic cells, macrophages or microglia. Agents that bind to and activate various TLRs, including any of TLR2, TLR3, TLR4, TLR7, TLR8, and TLR9, among others, are useful in the methods disclosed herein. In one embodiment, the agent is an unmethylated CpG oligonucleotide that binds to and activates TLR9. In another embodiment, the agent is poly I:C or another agent that binds to and activates TLR3. In another embodiment, the agent is gardiquimod, CL075, resiquimod, imiquimod, or another agent that binds to and activates TLR7 and/or TLR8. In yet other embodiments, the agent is MALP-2, which binds to and activates TLR2 or a nontoxic analog LPS, which binds to and activates TLR4.

Excitotoxic brain injury can be the result of a variety of disparate events. For example, the disclosed methods are suitable for protecting cells from injury or death due to epilepsy, traumatic brain injury and Alzheimer's disease, as well as stroke. Following administration, the agent binds to a TLR and induces cellular changes (for example, in the genomic program), such as inducing production of one or more neuroprotective cytokines, such as TGFβ, TNFa, and IFNβ.

To exert a protective preconditioning effect, the agent that binds to the TLR is administered prior to the excitotoxic event. For example, the agent can be administered to a subject identified as being at risk for an excitotoxic brain injury. In one exemplary application, the agent is administered to a subject prior to a surgical procedure, such as a surgical procedure involving the CNS or cardiovascular system. For example, such methods can be employed to protect a subject from excitotoxic brain injury resulting from surgical procedures involving arterial bypass, which are associated with an increased risk of excitotoxic brain injury, such as endarterectomy, pulmonary bypass and coronary artery bypass surgeries. Surgical interventions are typically non-recurring events; thus, the agent can be administered prior to the event in a single dose delivered prior to the start of the event. For optimal preconditioning effects, the agent is usually administered at least about 10 hours prior to the event (for example, surgery), and can be administered up to about 1 week prior to the event. Optionally, more than one doses of the agent are administered prior to the event.

To exert a treatment effect, the agent that binds to the TLR is administered following the excitotoxic event. For example, the agent can be administered to a subject identified as having suffered an excitotoxic brain injury. In one exemplary application, the agent is administered to a subject following an excitotoxic brain injury, for example at least 1 hour following the event. Optionally, more than one dose of the TLR ligand is administered following to the event.

Another aspect of the disclosure relates to methods of (1) protecting non-neural cells against ischemia and (2) treating non-neural cells that have suffered adverse effects due to ischemia, by systemically administering a TLR ligand. Typically, the TLR is expressed by a cell other than a cell of the central nervous system. For example, the non-neural cell can be a muscle cell (including a skeletal, smooth or cardiac muscle cell), a kidney cell, a liver cell, a spleen cell, a lung cell, an endothelial cell or a cell of the immune system (such as a dendritic cell, lymphocyte, macrophage or microglial cell). In certain cases, the ischemic event is associated with a surgical procedure, such as coronary artery bypass surgery. As discussed above, with respect to other preconditioning regimens, the TLR ligand is administered prior to the onset of ischemia. In contrast, for treatment, the TLR ligand is administered following the onset of ischemia. In an embodiment, the TLR ligand binds to TLR9, TLR3, TLR7 (and/or TLR8), TLR2 or TLR4. Exemplary agents include gardiquimod and gardiquimod derivatives, resiquimod and derivatives, CL057 and derivatives, CpG oligonucleotides, poly I:C and analogs thereof, imiquimod, MALP-2 and nontoxic LPS analogs, as well an TLR antibodies that activate the receptor.

Another aspect of the disclosure relates to methods of (1) protecting a transplanted organ against ischemia and (2) treating an organ transplant recipient that has suffered adverse effects due to ischemia, by systemically administering a TLR ligand. For example, organ donor subjects can receive the TLR ligand prior to removal of the organ, to protect the patient and the organ from adverse ischemic events. The harvested organ can also be contacted with the TLR ligand to protect or treat the organ from adverse ischemic events. In addition, the organ recipient can be administered a TLR ligand before or after the transplant to protect the patient and the organ from adverse ischemic events. In an embodiment, the TLR ligand binds to TLR9, TLR3, TLR7 (and/or TLR8), TLR2 or TLR4. Exemplary agents include gardiquimod and gardiquimod derivatives, resiquimod and derivatives, CL057 and derivatives, CpG oligonucleotides, poly I:C and analogs thereof, imiquimod, MALP-2 and nontoxic LPS analogs, as well an TLR antibodies that activate the receptor.

It will be apparent that the precise details of the methods or compositions described can be varied or modified without departing from the spirit of the disclosure. The examples are provided to illustrate certain particular features and/or embodiments. These examples should not be construed to limit the invention to the particular features or embodiments described. Each of the references cited herein is incorporated by reference.

Additional technical details are provided under the specific topic headings below.

### Terms

Unless otherwise explained, all technical and scientific terms used herein have the same meaning as commonly understood by one of ordinary skill in the art to which this disclosure belongs. Definitions of common terms in molecular biology may be found in Benjamin Lewin, Genes V, published by Oxford University Press, 1994 (ISBN 0-19-854287-9); Kendrew et al. (eds.), The Encyclopedia of Molecular Biology, published by Blackwell Science Ltd., 1994 (ISBN 0-632-02182-9); and Robert A. Meyers (ed.), Molecular Biology and Biotechnology: a Comprehensive Desk Reference, published by VCH Publishers, Inc., 1995 (ISBN 1-56081-569-8).

The singular terms "a," "an," and "the" include plural referents unless context clearly indicates otherwise. Similarly, the word "or" is intended to include "and" unless the context clearly indicates otherwise. The term "plurality" refers to two or more. It is further to be understood that all base sizes or amino acid sizes, and all molecular weight or molecular mass values, given for nucleic acids or polypeptides are approximate, and are provided for description. Although methods and materials similar or equivalent to those described herein can be used in the practice or testing of this disclosure, suitable methods and materials are described herein. The term "comprises" means "includes." The abbreviation, *"e.g."* is derived from the Latin *exempli gratia,* and is used herein to indicate a non-limiting example. Thus, the abbreviation "e.g." is synonymous with the term "for example."

In order to facilitate review of the various embodiments of this disclosure, the following explanations of specific terms are provided:

The term **"alkoxy"** refers to a substituted or unsubstituted alkoxy, where an alkoxy has the structure -O-R, where R is substituted or unsubstituted alkyl. In an unsubstituted alkoxy, the R is an unsubstituted alkyl. The term "substituted alkoxy" refers to a group having the structure -O-R, where R is alkyl which is substituted with a non-interfering substituent. "Lower alkoxy" refers to any alkoxy in which R is a lower alkyl. Particular examples of alkoxys include methoxy, ethoxy and propoxy groups.

The term "alkyl" refers to a cyclic, branched, or straight chain alkyl group containing only carbon and hydrogen, and unless otherwise mentioned contains one to twelve carbon atoms. This term is further exemplified by groups such as methyl, ethyl, n-propyl, isopropyl, isobutyl, t-butyl, pentyl, pivalyl, heptyl, adamantyl, and cyclopentyl. Alkyl groups can either be unsubstituted or substituted with one or more substituents, for instance, halogen, alkyl, alkoxy, alkylthio, trifluoromethyl, acyloxy, hydroxy, mercapto, carboxy, aryloxy, aryloxy, aryl, arylalkyl, heteroaryl, amino, alkylamino, dialkylamino, morpholino, piperidino, pyrrolidin-1-yl, piperazin-1-yl, or other functionality.

A **"CpG oligonucleotide"** or **"CpG ODN"** is a nucleotide molecule, typically between about 12 and 30 nucleotides in length and including at least one unmethylated cytosine-guanosine dinucleotide. Such molecules can bind to and activate TLR9. Generally, the unmethylated CpG dinucleotide is located at the interior of the nucleotide sequence rather than at an end. Unmethylated CpG dinucleotides are found throughout various genomes, including those of many bacteria and viruses. However, in the context of this disclosure, the CpG oligonucleotide is a synthetic (or isolated) nucleotide sequence. In some cases, the CpG oligonucleotide includes one or more nucleotides with a phosphorothioate modified backbone to increase stability of the CpG oligonucleotide *in vivo.*

A **"cytoprotective cytokine"** is a soluble protein (or glycoprotein) involved in the regulation of cellular proliferation and function that acts to preserve cellular function and prevent (or reduce) death of a cell in response to a stressful or otherwise aversive stimulus. Cytoprotective cytokines include transforming growth factor ß (TGF-ß), tumor necrosis factor α(TNFα), and type I interferons, such as interferon ß (IFNß). A "neuroprotective cytokine" is a cytoprotective cytokine that acts to preserve cellular function and reduce cell death in neural cells.

The phrase "excitotoxic injury" or "excitotoxic brain injury" refers to injury (including death), of neural cells, particularly neural cells of the brain, due to excessive stimulation of cell-surface receptors. Most commonly, excitotoxic injury is mediated through glutamate receptors, for example, by overactivation of N-methyl-d-aspartate (NMDA)-type glutamate receptors, resulting in excessive Ca²⁺ influx through the receptor's associated ion channel. Excitotoxic injury is believed to play a role in diverse conditions, including epilepsy, traumatic injury, and Alzheimer's disease.

The term **"hypoxia"** refers to a lack of oxygen. In a physiological context, the term hypoxia refers to an insufficiency of oxygen at a cellular, tissue or organismal level. Hypoxia can be caused by, for example, the reduction in partial pressure of oxygen (in the blood or in a tissue), inadequate oxygen transport (for example, due to a failure of oxygenated blood to reach a target tissue or cell), or the inability of the tissues to use oxygen. The term **"infarct"** refers to cell or tissue death due to a localized lack of oxygen (hypoxia).

Frequently, hypoxia is the result of "ischemia," the reduction in oxygenated blood flow to a target tissue or organ. An "ischemic event" is an event or occurrence that results in decreased blood flow to a cell, collection or group of cells, tissue, or organ. Ischemic events include vasoconstriction, thrombosis and embolism, resulting in reduced blood flow to a tissue or organ.

The term "lower alkyl" refers to a cyclic, branched or straight chain monovalent alkyl radical of one to five carbon atoms. This term is further exemplified by such radicals as methyl, ethyl, n-propyl, i-propyl, n-butyl, t-butyl, i-butyl (or 2-methylpropyl), cyclopropylmethyl, i-amyl, and n-amyl. Lower alkyl groups can also be unsubstituted or substituted, where a specific example of a substituted alkyl is 1,1-dimethyl propyl. Particular examples of lower alkyls are methyl, butyl and propyl (including isopropyl).

The term **"medicament"** is used interchangeably with the term **"pharmaceutical composition."** Such compositions are formulated for administration to human and/or animal (veterinary) subjects, and typically include one or more active component (such as one or more of the TLR ligands disclosed herein) as well as one or more additional components to facilitate administration to a subject, for the therapeutic or prophylactic treatment (prevention or reduction) of a condition or disorder. The additional components can include pharmaceutically acceptable carriers, buffers or excipients. Pharmaceutically acceptable carriers, buffers and so forth, are well known in the art, and are described, *e.g.,* in Remingtons Pharmaceutical Sciences, 19th Ed., Mack Publishing Company, Easton, Pennsylvania, 1995.

**A "neural cell"** is any cell in a lineage that originates with a neural stem cell and includes a mature neuron. Thus, the term neural cell includes neurons (nerve cells) as well as their progenitors regardless of their stage of differentiation. In the context of an adult brain, neural cells are predominantly differentiated neurons. In one example, neural cells include hippocampal neurons and cortical neurons. In contrast, a **"non-neural cell"** is a cell of a lineage other than a neural cell lineage, which is a lineage that does not culminate in the differentiation of a mature neuron. The non-neural cell may reside in the central nervous system (CNS), for example, in the brain (such as glial cells and immune system cells, such as B cells, dendritic cells, macrophages and microglia), or may exist in an organ outside the CNS, such as cardiac, skeletal or smooth muscle (a muscle cell), lung, intestine, liver (a hepatic cell) or kidney (a renal cell) and so forth. Non-neural cells include cells of the immune system (such as a dendritic cell, lymphocyte, macrophage or a microglial cell), regardless of whether they reside in the CNS or elsewhere in the body of the organism.

A **"preconditioning dose"** is a dose of an effective compound (such as a TLR ligand), or composition containing such a compound, that protects a cell against future injury or death due to an excitotoxic, ischemic or hypoxic event. The dosage of the effective compound or composition varies from compound to compound and between species. A suitable preconditioning dose for any compound can be determined empirically.

**"Prophylactic"** treatment refers to the treatment of a subject prior to the full manifestation of an event, condition or disease for the purpose of preventing or reducing the symptoms, signs or consequences of the event, condition or disease. Thus, in the context of the present disclosure, prophylactic treatment of an excitotoxic injury or hypoxia refers to the treatment of a subject prior to the occurrence of an excitotoxic or hypoxic event (that is, prior to a first excitotoxic or hypoxic event, or prior to a subsequent excitotoxic or hypoxic event, or prior to the completion or culmination of an ongoing or recurrent excitotoxic or hypoxic event) and prior to the completion of the natural consequences and/or sequelae of the event.

The term **"protect"** with respect to an excitotoxic or hypoxic event refers to the ability of composition or treatment regimen to prevent, reduce in severity, or otherwise lessen the effects of an excitotoxic or hypoxic event at a cellular, tissue or organismal level. Methods for measuring severity of effects of an excitotoxic or hypoxic event include neurological, including behavioral, indicia (e.g., ascertainable via neurological examination of a subject) as well as by evaluation of cellular and metabolic parameters, for example, by Computed Axial Tomography (CT scan, CAT scan); Magnetic Resonance Imaging (MRI scan, MR scan); Carotid Ultrasound, including Transcranial Doppler (TCD); Cerebral Angiography: (Cerebral arteriogram, Digital subtraction angiography [DSA]); Computed Tomographic Angiography: (CT-angiography, CT-A, CTA); Magnetic Resonance Angiography (MRA) and/or other diagnostic procedures known to those of ordinary skill in the art.

A subject is at **"risk for a cytotoxic insult"** or at **"risk for an excitotoxic, ischemic or hypoxic event"** if there is an increased probability that the subject will undergo an excitotoxic, ischemic or hypoxic event relative to the general population. Accordingly, risk is a statistical concept based on empirical and/or actuarial data. Commonly, risk can be correlated with one or more indicators, such as symptoms, signs, characteristics, properties, occurrences, events or undertakings, of a subject. For example, with respect to risk of stroke, indicators include but are not limited to high blood pressure (hypertension), atrial fibrillation, transient ischemic events, prior stroke, diabetes, high cholesterol, angina pectoris, and heart disease. More generally, risk indicators for hypoxic events include surgery, especially cardiovascular surgeries, such as endarterectomy, pulmonary bypass surgery or coronary artery bypass surgery. Additional risk factors or indicators include non-medical activities, such as motorcycle riding, contact sports and combat. Other risk factors are discussed herein, and yet more can be recognized by those of ordinary skill.

The term **"stroke"** refers to an interruption of the blood supply to any part of the brain. A stroke can be due to an ischemic event (for example, occlusion of a blood vessel due to a thrombus or an embolism) or hemorrhage (for example, of a cerebral blood vessel).

A **"subject"** is a living multi-cellular vertebrate organism, a category that includes both human and veterinary subjects, including human and non-human mammals.

The modifiers **"systemic**" and **"systemically"** are used in reference to administration/administering of a composition to indicate that administration results in the composition contacting cells and/or tissues at one or more sites at a distance to the site of administration, including cells and/or tissues of an organ or body part that is not the organ or body part into which the composition is directly administered. Most commonly, systemic administration involves introducing the composition directly or indirectly into the circulatory system of the organism. Thus, intravenous administration is one method of systemic administration of a composition. Additionally, a composition can be systemically administered by introducing the composition into a site that indirectly results in the composition being introduced into (either by diffusion or an active transport process) the circulatory system of the organism. Thus, intranasal, oral, transdermal, subcutaneous, intramuscular, intravenous, intrathecal and intraperitoneal routes can all be systemic administration of the composition. The term systemic is used to distinguish the administration route from methods that result in a composition being retained in close proximity (for example, within the same tissue or organ) to the site of introduction.

A **"Toll-like receptor"** or **"TLR"** is a type I transmembrane protein which acts as a pattern recognition receptor (PRR). Toll-like receptors play a role in innate immunity, for example, by recognizing conserved microbial structures or Pathogen-Associated Molecular Patterns (PAMP). Thirteen TLRs (named TLR1 to TLR13) have been identified. However, equivalents of certain TLR found in humans are not present in all mammals. For example, a gene coding for a protein analogous to TLR10 in humans is present in mice, but appears to have been damaged by a retrovirus. On the other hand, mice express TLRs 11, 12, and 13, none of which are represented in humans.

Nucleic acid sequences that encode human TLRs, and corresponding protein sequences are publically available, e.g., as shown in Table 1 (all Genbank numbers referred to herein are incorporated by reference for the sequence available on November 13, 2009). Naturally occurring and artificial ligands of several TLRs have been characterized. Exemplary ligands are shown in Table 1. A TLR ligand is said to "activate" a TLR receptor if the ligand binds to the receptor, and such binding results in the initiation of one or more signaling events, such as translocation or phosphorylation of the TLR receptor and/or other signaling molecules. Thus, TLR agonists can be used in addition to or in place of particular TLR ligands described herein.

**Table 1: Exemplary TLR sequences and ligands.**

| | **Genbank nucleic acid sequences** | **Genbank protein sequences** | **Ligands** | **Cell types** |
|---|---|---|---|---|
| **TLR1** | U88540; AB445617.1; BC141321.1 | AAC34137; AB445617; AAI41320.1 | triacyl lipopeptides such as Pam(3)Cys-Ser-(Lys)(4) | monocytes/macrophages; a subset of dendritic cells; B lymphocytes |
| **TLR2** | U88878; NM 011905.3 | AAC34133.1 ; AAD49335.1 | multiple glycolipids, lipopeptides, and lipoproteins; lipoteichoic acid; HSP70; zymosan; MALP-2 | monocytes/macrophages; myeloid dendritic cells; mast cells |
| **TLR3** | U88879; NG_007278.1; NM_126166.4 | AAC34134.1; BAG55028.1; AAH99937.1 | poly I:C; poly(I:C₁₂U); dsRNA (a viral product) | dendritic cells; B lymphocytes |
| **TLR4** | U88880; NG_011475 | AAC3413 5.1; CAH72619.1; CAH72618.1; AAD29272.1 | lipopolysaccharides (LPS); peptidoglycan fragments (glycopeptides); several heat shock proteins; fibrinogen; heparan sulfate fragments; hyaluronic acid fragments | monocytes/macrophages; myeloid dendritic cells; mast cells; intestinal epithelium |
| **TLR5** | AB060695.1; BC125247 | ACM69034.1; BAB43955.1; AAI2 5248.1; NP_058624.2 | bacterial flagellin | monocyte/macrophages; a subset of dendritic cells; intestinal epithelium |
| **TLR6** | AB020807; EU195556.1; NM 011604.3 | ABY67133.1; NP_035734.3 | multiple diacyl lipopeptides | monocytes/macrophages; mast cells; B lymphocytes |
| **TLR7** | AF245702; AK313858 | AAF78035.1; BAG36586.1; CAM 14953.1 | gardiquimod; single stranded RNA (such as viral RNA); bropirimine; loxoribine; imidazoquinoline; imiquimod; resiquimod; CL075 | monocytes/macrophages; plasmacytoid dendritic cells; B lymphocytes |
| **TLR8** | AF245703; BC132054.1 | AAF78036.1; CAM14949.1 | single stranded RNA (such as viral RNA); resiquimod; CL075 | monocytes/macrophages; a subset of dendritic cells; mast cells |
| **TLR9** | AB045181.1; AF245704; AF259262; AF259263 | AAF78037.1; BAB19260.1; AAK28488.1 | CpG oligonucleotides; unmethylated CpG DNA (such as those found in the genome of bacteria and viruses) | monocytes/macrophages; plasmacytoid dendritic cells; B lymphocytes |
| **TLR10** | AF296673; AB445680.1; NM_001146035.1 | AAK26744.1; BAG55077.1; NP_001139507 | | monocytes/macrophages; B lymphocytes |
| **TLR11** | FJ539013.1; AY510704.1 | AAS37672.1 ; ACL80330.1 | Profilin | monocytes/macrophages; liver cells; kidney; bladder epithelium |
| **TLR12** | NM_001108682.1; NM_205823.2 | NP_001102152.1; AAS37673.1 | | |
| **TLR13** | NM_205820.1 | AAS37674.1 | | |

"Treatment" refers to the treatment of a subject following the full manifestation of an event, condition or disease for the purpose of treating or reducing the symptoms, signs or consequences of the event, condition or disease after they occur. Thus, in the context of the present disclosure, treatment of an excitotoxic injury, hypoxia, or ischemia refers to the treatment of a subject following the occurrence of an excitotoxic, hypoxic, or ischemic event. However, one skilled in the art will appreciate that 100% treatment is not required in order to consider a treatment to be effective. For example, a notable reduction in the adverse effects associated with an excitotoxic injury, hypoxia, or ischemia can be sufficient. For example, a therapeutic composition can decrease the sign or symptom by a desired amount, for example by at least 20%, at least 50%, at least 80%, at least 90%, at least 95%, at least 98%, or even at least 100%, as compared to the sign or symptom in the absence of the TLR ligand.

### Treatment

Exposure of cells to therapeutic levels of TLR ligand following a cytotoxic insult such as excitotoxic events and hypoxia (e.g., due to ischemia), can be used to treat a variety of cell and tissue types, including neural cells, muscle cells *(e.g.,* skeletal as well as cardiac muscle cells), kidney cells, lung cells, intestinal cells ,and liver cells. Treatment in the brain (e.g., neural cells) and other organs can be produced following exposure to therapeutic levels of TLR ligand. This effect is dependent on *de novo* protein synthesis, and involves changes in genomic programming associated with inflammation.

Following administration of a suitable TLR ligand (such as a CpG oligonucleotide, gardiquimod, R848, CL075, imiquimod, poly I:C or other agent that activates a TLR), treatment of adverse consequences due to excitotoxic, ischemic, and/or hypoxic injury typically begins within about 1-24 hours of such an event and can last for up to several weeks, or more. In addition, additional therapeutic benefits can be extended by repeated administration of the TLR ligand.

Interferons (IFNs) are involved in treatment of excitotoxic injury, ischemia and hypoxia. IFNs are a family of cytokines comprised of type I (IFNα and IFNß) and type II (IFNy) IFNs. First characterized based on anti-viral properties, type I IFNs have many immunomodulatory functions. Generally, IFNα/ß are associated with anti-inflammatory cytokines (Shnyra et al., J. Immunol. 160:3729-3736, 1998).

IFNß has been shown to improve stroke outcome following systemic administration in animal models (Veldhuis et al., J. Cereb. Blood Flow Metab. 23:1029-1039, 2003; Liu et al., Neurosci Lett. 327:146-148, 2002). The mitigating role of IFNß in stroke is primarily due to its anti-inflammatory properties that reduce cell infiltration into the affected tissue via regulation of matrix metalloproteinase-9. In addition, IFNß has been shown to decrease reactive oxygen species, suppress inflammatory cytokines (Hua et al., J. Neurochem. 83:1120-1128, 2002) and promote cell survival (Barca et al., J. Neuroimmunol., 139:155-159, 2003), functions that contribute to improved outcome following stroke.

The basis for some of IFNα/ß's regulatory functions lies in their action as a facilitator of expression of other IFN-inducible proteins known as IFN regulatory factors (IRFs), that in turn transactivate additional IFN-inducible genes (Taniguchi et al., Annu. Rev. Immunol. 19:623-655, 2001). IRFs constitute a family of transcription factors whose functions in some instances are distinct and independent of one another, while in others, appear to be interdependent (Taniguchi and Takaoka, Curr. Opin. Immunol. 14:111-116, 2002). For example, IRF3 binding to the interferon stimulated response element (ISRE) induces IFNß which is involved in the early stages of preconditioning. The ability of IRF3 to transactivate IFNß in this scenario depends on NFκB as well. Interaction between these two transcription factors is extensive; IRF3-NFκB complexes have been shown to interact not only at the ISRE but at κB sites as well (Wietek et al., J. Biol. Chem. 278:50923-50932, 2003; Leung et al., Cell 118:453-464, 2004). Furthermore, many genes contain both IRSE and _{K}B sites within their promoter regions and depend upon interaction between the two factors for transcription initiation *(*Genin et al., J. Immunol. 164:5352-5361, 2000). IRF3 is induced by agents that activate TLRs and is likely to mediate the treatment effects of TLR ligands.

The present disclosure provides methods for treating cells by systemically administering an agent that binds to a TLR and thereby induces changes in the genomic program of certain cells, for example, as described above by altering the nature and amount of cytokines produced. For example, an agent that activates a TLR *(e.g.,* a poly I:C) can be administered to a subject using a systemic administration route. Binding of the agent to TLR expressed on the surface of target cells, for example, certain immune system cells, including B cells, dendritic cells (DC), macrophages, and microglial cells, results in genomic reprogramming and in the case of the above mentioned immune cells, can induce an alteration in the cytokine secretion profile, including the induction of cytoprotective cytokines, such as type I interferons (e.g., IFN-a, IFN-ß), TGF-ß and/or IL-10.

### Selecting subjects that have suffered a cytotoxic insult

In particular examples, the methods disclosed herein are applicable to any cell types that can suffer from (e.g., have adverse or undesirable consequences) to excitotoxic, ischemic and/or hypoxic injury. For example, neural cells (including, e.g., hippocampal neurons and cortical neurons), muscle cells (including cardiac and striated muscle cells), hepatic cells, lung cells, intestinal cells, and renal cells can be treated for injury by administering a TLR ligand or agonist (such as a TLR1, TLR2, TLR4 (but not LPS) TLR3, TLR5, TLR6, TLR7, TLR8, or TLR9, and in a specific example a TLR3 or TLR 7 ligand) following the occurrence of an excitotoxic, ischemic or hypoxic event. Thus, a TLR ligand can be administered to a subject that has been identified as having suffered or experienced (e.g., diagnosed with), or suspected of having suffered or experienced, an excitotoxic, ischemic and/or hypoxic event.

Excitotoxic injury results from excessive stimulation of cells (typically neural cells in the CNS) by certain neurotransmitter (e.g., glutamate) receptors. Excitotoxic injury can be a result of a condition that causes excessive chemical or electrical activity in the brain or it can be a result of conditions that cause a decrease in inhibitory or regulatory functions of the brain. Excitotoxic injury in the brain is associated with a variety of conditions with disparate etiologies and symptoms, including epilepsy, traumatic brain injury and Alzheimer's disease. For example, traumatic brain injury (regardless of its cause) frequently involves an excitotoxic (and can also include) a hypoxic component. Thus, in some examples subjects who have previously suffered from such conditions are selected for treatment according to the methods disclosed herein.

Hypoxia is typically associated with ischemic events in the CNS or elsewhere in the cardiovasculature, (such as cerebrovascular ischemia, or stroke, myocardial ischemia due to narrowing or blockage of the vessels of the heart, iatrogenic ischemia, due to surgical procedures, mesenteric ischemia (where blood supply to the intestine and mesentery are impaired), and the like). In addition, hypoxia can occur *in utero* due to conditions such as inadequate placental function (for example, due to abrupio placentae), preeclamptic toxicity, prolapse of the umbilical cord, or complications from anesthetic administration. Additionally, injury by some hypoxic events (such as strokes) involves an excitotoxic component as well as a hypoxic component. Such hypoxic or ischemic injuries can occur to any cell in the body, such as a muscle cell, immune cell, intestinal or mesenteric cell, kidney cell, or lung cell. Thus, various cardiovascular signs and symptoms, such as atrial fibrillation, angina pectoris, hypertension, transient ischemic episodes and prior stroke, are all indicators that a subject has previously suffered from a hypoxic or ischemic event, and can be used to select a subject for administration of a TLR ligand. Elderly patients or patients presenting with abdominal pain or weight loss with other risk factors (such as colitis, diabetes, peripheral arterial insufficiency, hypertension, pulmonary edema or prior abdominal surgery) can be indicators that a subject has suffered mesenteric ischemia.

### Preconditioning

Exposure of cells to subthreshold levels (that is, at a level below that which causes injury) of a stressful (e.g., cytotoxic) stimulus can induce tolerance to subsequent events that would otherwise result in injury. This effect is termed preconditioning and is relevant to preventing or reducing injury due to cytotoxic insult such as excitotoxic events and hypoxia (e.g., due to ischemia) in a variety of cell and tissue types, including neural cells, muscle cells (e.g., skeletal as well as cardiac muscle cells), kidney cells, lung cells, intestinal cells, and liver cells.

Preconditioning in the brain (e.g., neural cells) and other organs can be produced following exposure to a subthreshold level of an otherwise toxic stimulus. For example, brief exposure to ischemia and administration of a sub-toxic dosage of lipopolysaccharide (LPS) has been shown to elicit a protective response to subsequent ischemic events. This effect is dependent on *de novo* protein synthesis, and involves changes in genomic programming associated with inflammation. Following administration of a suitable preconditioning agent (such as poly I:C, a CpG oligonucleotide, gardiquimod, CL075, R848, imiquimod, or other agent that activates a TLR), protection against excitotoxic and/or hypoxic injury typically begins within about 10-12 hours and lasts for up to several weeks, or more. In addition, protection can be extended by repeated administration of the agent.

The activation of inflammatory pathways is involved in preconditioning against excitotoxic injury, ischemia and hypoxia. For example, TNFα and its downstream signaling mediator, ceramide, are involved in achieving a preconditioning effect, and blockade of TNFα (with a soluble TNF receptor or fragment thereof) prevents the protective effect of preconditioning. Thus, proximal members of the TNFα pathway, namely TNFα and its receptors, TNFRI (p55) and TNFR2 (p75), as well as sphingomyelin-based second messengers such as ceramide, are likely mediators of the protective effects of TNFα in LPS preconditioning. TNFα-activation of NF_{-κ}B may also be involved, as inflammatory molecules regulated by NF-κB, such as superoxide dismutase (SOD), have been shown to be involved in preconditioning (Bordel et al., J. Cereb. Blood Flow Metab. 20:1190-1196, 2000).

Interferons are also involved in cytoprotection against excitotoxic injury, ischemia and hypoxia. IFNs are a family of cytokines comprised of type I (IFNa and IFNß) and type II (IFNγ) IFNs.

The basis for some of IFNα/ß's regulatory functions lies in their action as a facilitator of expression of other IFN-inducible proteins known as IFN regulatory factors (IRFs), that in turn transactivate additional IFN-inducible genes (Taniguchi et al., Annu. Rev. Immunol. 19:623-655, 2001). IRFs constitute a family of transcription factors whose functions in some instances are distinct and independent of one another, while in others, appear to be interdependent (Taniguchi and Takaoka, Curr. Opin. Immunol. 14:111-116, 2002). For example, IRF3 binding to the interferon stimulated response element (ISRE) induces IFNß which is involved in the early stages of preconditioning. The ability of IRF3 to transactivate IFNß in this scenario depends on NFκB as well. Interaction between these two transcription factors is extensive; IRF3-NFκB complexes have been shown to interact not only at the ISRE but at κB sites as well (Wietek et al., J. Biol. Chem. 278:50923-50932, 2003; Leung et al., Cell 118:453-464, 2004). Furthermore, many genes contain both IRSE and κB sites within their promoter regions and depend upon interaction between the two factors for transcription initiation (Genin et al., J. Immunol. 164:5352-5361, 2000). IRF3 and IRF7 are induced by agents that activate TLRs and are likely to mediate the cytoprotective effects of preconditioning agents.

Preconditioning involves a fundamental change in the genomic program or response (that is, the pattern of gene expression produced in response) to excitotoxic, ischemic and/or hypoxic injury that shifts the outcome from cell death to cell survival (Stenzel-Poore et al., The Lancet 362:1028-1037, 2003). This change in gene expression, or genomic reprogramming, in response to cytotoxic insults, such as excitotoxic, ischemic and/or hypoxic events, may involve a pronounced suppression of gene expression (for example, of inflammatory cytokines, and certain ion channels and channel regulators, e.g., **K**⁺ and Ca⁺⁺ channels, such as glutamate receptors), which is ordinarily injurious. Such suppression contrasts sharply with the upregulation of mRNA by excitotoxic, ischemic and/or hypoxic events without preconditioning. This change is not simply the lack of a response, but rather a reprogramming of the genomic response that involves the downregulation of genes that control metabolism, cell-cycle regulation, and, in neural cells, ion-channel activity. Additionally, in certain cells of the immune system, preconditioning elicits a shift from pro-inflammatory to anti-inflammatory cytokines.

Preconditioning in macrophages, leading to suppression of specific cytokines and inflammatory molecules, involves attenuation of NF_{-κ}B and AP-1 and enhanced expression of the signaling mediators, IRAK-M and SOCS-1 (Kobayashi et al., Cell 110:191-200, 2002; Nakagawa et al., Immunity 17:677-687, 2002; Kinjyo et al., Immunity 17:583-591, 2002). Similar genomic reprogramming is also likely to be involved in preconditioning in cardiac tissue (Meng et al., Am. J. Physiol. 275:C475-483, 1998), although the specific genes can differ (e.g., HSP70, c-jun, c-fos).

The present disclosure provides methods for preconditioning cells by systemically administering an agent that binds to a TLR and thereby induces changes in the genomic program of certain cells, for example, as described above by altering the nature and amount of cytokines produced. For example, an agent that activates a TLR (e.g., poly I:C, a CpG oligonucleotide, gardiquimod, R848, or CL075) can be administered to a subject using a systemic administration route. Binding of the agent to TLR expressed on the surface of target cells, for example, certain immune system cells, including B cells, dendritic cells (DC), macrophages, and microglial cells, results in genomic reprogramming and in the case of the above mentioned immune cells, can induce an alteration in the cytokine secretion profile, including the induction of cytoprotective cytokines, such as TNF-α, type I interferons (e.g., IFN-α, IFN-ß) and/or TGF-ß.

### Selecting subjects at risk for cytotoxic insult

In some examples, the methods disclosed herein are applicable to any cell types susceptible to excitotoxic, ischemic and/or hypoxic injury, which are amenable to preconditioning. For example, neural cells (including, e.g., hippocampal neurons and cortical neurons), muscle cells (including cardiac and striated muscle cells), hepatic cells, lung cells, intestinal or mesenteric cells, and renal cells can be protected against injury and death by administering a TLR ligand (such as TLR1, TLR2, TLR3, TLR4, TLR5, TLR6, TLR7, TLR8, or TLR9 ligand) prior to the occurrence of an excitotoxic, ischemic or hypoxic event. Thus, a preconditioning agent (e.g., TLR ligand) can be administered to a subject that has been identified as having (e.g., diagnosed with) one or more risk factors indicative of an increased likelihood, relative to the general population or to a subject without the risk factor, of having an excitotoxic, ischemic and/or hypoxic event.

As discussed above, excitotoxic injury results from excessive stimulation of cells (typically neural cells in the CNS) by certain neurotransmitter (e.g., glutamate) receptors. Subjects as risk for having an excitotoxic injury in the future can be due to the presence of a condition that causes excessive chemical or electrical activity in the brain or a condition that causes a decrease in inhibitory or regulatory functions of the brain. Thus, in some examples the subject at risk for future excitotoxic injury has epilepsy, a traumatic brain injury or Alzheimer's disease. In addition to medical indications such as epilepsy or Alzheimer's disease, non-medical indicators of risk, pertaining to behaviors or activities that are statistically associated with an increased likelihood of injuries that can include an excitotoxic component. For example, traumatic brain injury (regardless of its cause) frequently involves an excitotoxic (and can also include) a hypoxic component. Thus, participation in activities that increase the risk of traumatic brain injury are indicators that can be used to select a subject for administration of a TLR ligand. Such activities include, for example, motorcycle riding, motor vehicle racing, skiing, contact sports (such as, football, hockey, rugby, soccer, lacrosse, martial arts, boxing and wrestling), and the like. Additionally, impacts or wounds resulting from gunshot or explosives frequently cause traumatic brain injury. Accordingly, activities that are associated with an increased risk of gunshot wounds or injury caused by explosive devices (for example, in combat situations) are an indicator of risk that can be used to select a subject for treatment according to the methods disclosed herein.

Hypoxia is typically associated with ischemic events in the CNS or elsewhere in the cardiovasculature, (such as cerebrovascular ischemia, or stroke, myocardial ischemia due to narrowing or blockage of the vessels of the heart, iatrogenic ischemia, due to surgical procedures, and the like). In addition, hypoxia can occur *in utero* due to conditions such as inadequate placental function (for example, due to abrupio placentae), preeclamptic toxicity, prolapse of the umbilical cord, or complications from anesthetic administration. Additionally, injury by some hypoxic events (such as strokes) involves an excitotoxic component as well as a hypoxic component.

Thus, various cardiovascular signs and symptoms, such as atrial fibrillation, angina pectoris, hypertension, transient ischemic episodes and prior stroke, are all indicators of risk (or risk factors) that can be used to select a subject for administration of a TLR ligand. Similarly, surgical procedures, especially those specifically involving the cardiovascular system, such as endarterectomy, pulmonary bypass and coronary artery bypass surgeries, are indicators of risk that can be used to select a subject for administration of a preconditioning agent.

### TLR Ligands

Agents that bind to and activate TLRs (including TLR ligands and agonists) can also be used in the methods herein. At least ten TLRs have been reported in humans (Janeway and Medzhitov, Annu. Rev. Immunol. 20:197-216, 2002). In some examples, one type of TLR ligand is used. In other examples, two or more different TLR ligands are administered to the subject, such as a TLR7/8 and TLR9 ligand, or a TLR3 ligand and a TLR7/8 ligand. One skilled in the art will appreciate that other therapeutic agents can be administered in combination with the TLR ligand(s), such as Tpa. In addition to TLR ligands, agents that activate interferon regulatory factor 3 (IRF3) can be used (for example see US Patent Application Publication No. 20060057104).

TLRs are expressed on the surface of cells in a wide variety of tissues, including brain, heart, intestine, kidney, liver, lung, skeletal muscle, spleen and thymus. In addition overlapping subsets of TLRs are expressed on different cells of the immune system. For example, TLR9 is highly expressed on human dendritic cells and B cells, whereas TLR2 is most highly expressed on monocytes, as is TLR4. TLR1 is expressed well on monocytes, dendritic cells and B cells, as well as on NK cells and T cells. TLR5 is also expressed on T cells, NK cells and monocytes, but little expression is seen on B cells or monocytes. TLR6 is expressed on all of the above cell lineages, with expression being highest on B cells. TLR7 is expressed on monocytes, B cells and dendritic cells, with highest expression in dendritic cells.

A common pathway in all the TLRs is the ability to induce NFκB, which subsequently leads to the transcription of various cytokines, chemokines and cell surface molecules (Andreakos et al., Immunol. Rev. 202:250-265, 2004). This induction of NF-κB is involved in establishment of therapeutic benefits using the regimens provided herein. Thus, any agent that activates or stimulates TLRs and that are suitable for administration *in vivo* can be used in the methods provided herein. Exemplary non-limiting classes of TLR ligands or agonists include those agents that modulate or bind to TLR protein or nucleic acid and confer signaling in a cell *(in vitro, ex vivo or in vivo)* by agonizing expression of or activity of intracellular TLR-mediated signaling pathway mediators, such as polypeptides, nucleic acid and small molecules. Additional non-limiting examples include antibodies (e.g., with two heavy chains and two light chains). Antibodies can bind to extracellular domain of TLRs. Antibodies that act as TLR agonists can be used in the disclose methods, and can be mammalian, such as a primate or fully or partially human, e.g., humanized or primatized antibody. Antibodies include monoclonal, polyclonal and mixtures thereof. Antibodies further include functional (e.g., antigen binding) subsequences (fragments) that bind to and activate TLR receptors, such as Fab, Fab', F(ab')₂, Fv, Fd, single-chain Fv (scFv), disulfide linked Fv, light chain variable (VL) and heavy chain variable (VH) forms. Specific non-limiting examples of TLR antibodies include the TLR4 antibody agonist 5D24.D4 that mimics the action of natural TLR ligands.

Exemplary TLR ligands that can be used in the disclosed methods are provided above in Table 1. Additional information on some of the ligands or agonists is provided below.

### TLR 2 ligands

TLR2 is involved in the recognition of a wide array of microbial molecules representing broad groups of species such as Gram-positive and Gram-negative bacteria, as well as mycoplasma and yeast. In one exemplary embodiment, a composition containing one or more TLR2 ligands (and in some examples with one or more other TLR ligands) is administered to a subject for the purpose of (1) treating one or more cells (or cell types, or tissues, or organs) following a or (2) preconditioning one or more cells (or cell types, or tissues, or organs) to protect against a subsequent, cytotoxic insult, such as an excitotoxic injury, ischemia or hypoxia.

Specific exemplary ligands that bind to activate TLR2 include macrophage-activating lipopeptide-2 (MALP-2) and derivatives thereof; zymosan (an insoluble preparation of yeast cell that activates macrophages via TLR2); lipoproteins (such as FSL1, a diacylated synthetic lipoprotein derived from *Mycoplasma salivarium* similar to MALP-2; Pam3CSK4, a synthetic tripalmitoylated lipopeptide (LP) that mimicks the acylated amino terminus of bacterial LPs; and Pam2CSK4, a synthetic diacylated LP), peptidoglycans (PGN) (such as PGN from *B. subtilis, E. coli,* or *S. aureus),* lipoteichoic acids (LTA), lipoglycans (such as lipoarabinomannans (LAM) and lipomannans (LM)), lipopolysaccharide from *P. gingivalis.* Mycobacterial ligands for TLR2 or the heterodimers TLR2/1 and TLR2/6 include the mycobacterial p 19 lipoprotein arabinosecapped Lipoarabinomannan (LAM), PIM, lipomannan and trehalose dimycolate. MALP-2 and its derivatives, such as S-[2,3-bispalmitoyloxy-(2R)-propyl]-R-cysteinyl-amido-monomethoxylpolyethyleneglyvcol, are TLR 2/6 agonists, that can also be used in the disclosed methods.

### TLR 3 ligands

In one exemplary embodiment, a composition containing one or more TLR3 ligands (and in some examples with one or more other TLR ligands) is administered to a subject for the purpose of (1) treating one or more cells (or cell types, or tissues, or organs) following a or (2) preconditioning one or more cells (or cell types, or tissues, or organs) to protect against a subsequent, cytotoxic insult, such as an excitotoxic injury, ischemia or hypoxia.

TLR3 recognizes double-stranded RNA (dsRNA), a molecular pattern associated with viral infection. Exemplary TLR3 ligands include dsRNA or a synthetic analog thereof, such as polyinosine-polycytidylic acid (poly(I:C)) and analogs thereof, such as poly(I:C₁₂U). Other exemplary agents that can be used to activate TLR3 include the TLR3 agonists stathmin or stathmin-like proteins such as SCG-10, SCLIP or RB3 (see for example WO 2007/089151, herein incorporated by reference as to these protein sequences).

### TLR4 ligands

In one exemplary embodiment, a composition containing one or more TLR4 ligands (and in some examples with one or more other TLR ligands) is administered to a subject for the purpose of (1) treating one or more cells (or cell types, or tissues, or organs) following a or (2) preconditioning one or more cells (or cell types, or tissues, or organs) to protect against a subsequent, cytotoxic insult, such as an excitotoxic injury, ischemia or hypoxia.

TLR4 is the receptor for Gram-negative lipopolysaccharide (LPS) and lipid A, its toxic moiety. An exemplary TLR4 ligand is lipopolysaccharide R515 (LPS)]. LPS for commercial sources can be used in the methods provided herein, for example from Sigma Aldrich Co. (St. Louis, MO) and Invivogen. Other known TLR4 ligands are RSV (respiratory syncytial virus) F protein, a prominent component of the viral envelope, and taxol, which acts as an LPS mimetic signaling via TLR4. Other exemplary agents that can be used to activate TLR4 include the TLR4 agonists, such as antibodies that mimic such ligands and activate TLR4 (such as the monoclonal antibody 5D24.D4, see Cohen et al. Hybridoma, 24:27-35, 2005, herein incorporated by reference).

### TLR5 ligands

In one exemplary embodiment, a composition containing one or more TLR5 ligands (and in some examples with one or more other TLR ligands) is administered to a subject for the purpose of (1) treating one or more cells (or cell types, or tissues, or organs) following a or (2) preconditioning one or more cells (or cell types, or tissues, or organs) to protect against a subsequent, cytotoxic insult, such as an excitotoxic injury, ischemia or hypoxia.

TLR5 recognizes flagellin, the major component of the bacterial flagellar filament, from both Gram+ and Gram- bacteria. Flagellin purified from *B. subtilis* (Gram+) and S. *typhimurium* (Gram-) bacteria or recombinant flagellin can be used and are commercially available, for example from Invivogen.

### TLR7/8 ligands

In one exemplary embodiment, a composition containing one or more TLR7/8 ligands (and in some examples with one or more other TLR ligands) is administered to a subject for the purpose of (1) treating one or more cells (or cell types, or tissues, or organs) following a or (2) preconditioning one or more cells (or cell types, or tissues, or organs) to protect against a subsequent, cytotoxic insult, such as an excitotoxic injury, ischemia or hypoxia.

TLR7 and TLR8 are involved in the response to viral infection. They recognize GU-rich short single-stranded RNA as well as small synthetic molecules such as imidazoquinolines and nucleoside analogues. TLR7 ligands include CL264 (Adenine analog), Gardiquimod™ (imidazoquinoline compound), imiquimod (imidazoquinoline compound), polyuridylic acid, and Loxoribine (guanosine analogue). TLR8 ligands include ssRNAs and TLR 7/8 ligands include CL075 (thiazoloquinoline compound), CL097 (water-soluble R848, imidazoquinoline compound), Poly(dT) (thymidine homopolymer phosphorothioate ODN) and R848 (resiquimod, an imidazoquinoline compound).

Imiquimod (and other imidazoquinoline compounds, such as resiquimod (also known as R-848)) bind to and activate TLR7 in mice, and TLR7 and TLR8 in humans (Hemmi et al., Nat Immunol., 3:196-200, 2002; Jurk et al., Nat Immunol. 3:499, 2002). In some examples, the chemical structure of imiquimod is:

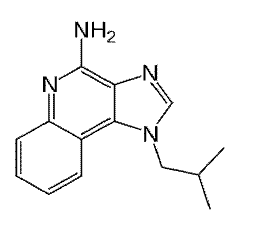

Previously, these compounds have been used as antiviral and antitumour agents, typically at doses ranging between 0.25 and 5 mg/kg. For the methods provided herein exemplary doses of imiquimod can range from about 0.002 mg/kg (such as 0.005 mg/kg, 0.008 mg/kg, about 0.01 mg/kg, about 0.02 mg/kg, about 0.05 mg/kg, about 0.1 1 mg/kg, about 0.5 mg/kg, or about 1 mg/kg) to about 2 mg/kg in primates, such as about 0.9 mg/kg). Administration of imiquimod (and/or other imidazoquinoline compounds or derivative(s) that bind to and activate TLR7/8) to a subject at risk of an excitotoxic injury, ischemia and/or hypoxia, prior to such a cytotoxic insult protects against cell injury and death. Thus, imiquimod (and related compounds or derivatives) can be used as an alternative to, or in combination with CpG oligonucleotides as preconditioning agents. In addition, administration of imiquimod (and/or other imidazoquinoline compounds or derivative(s) that bind to and activate TLR7/8) to a subject who has suffered an excitotoxic injury, ischemia and/or hypoxia, following such a cytotoxic insult treats cell injury and death. Thus, imiquimod (and related compounds or derivatives) can be used as an alternative to, or in combination with other TLR ligands, such as poly I:C.

Another exemplary TLR7/8 ligand is Gardiquimod. In some embodiments, the chemical structure of Gardiquimod or Gardiquimod derivative is:

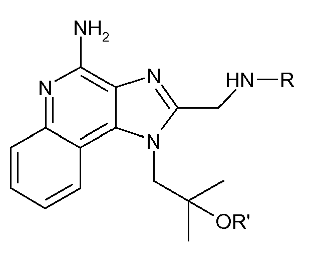

where R is an aliphatic group comprising 1-5 carbon atoms, and where R' is selected from hydrogen or an aliphatic group comprising 1-5 carbon atoms. Aliphatic groups are unbranched or branched carbon chains, and may be saturated (all single bonds) or unsaturated (one or more double or triple bonds). Specific examples of aliphatic groups include alkyl, alkenyl, and alkynyl groups. In another embodiment R' is selected from CH₂CH₂OH, CH₂CH(CH₃)OH or CH₂C(CH₃)₂OH.

In other examples, the structure of Gardiquimod is:

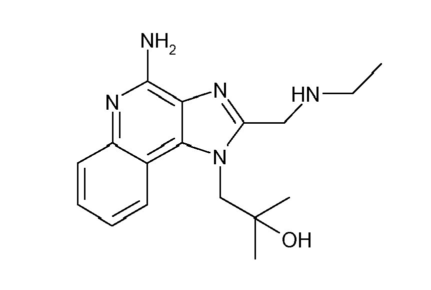

Exemplary therapeutic doses of Gardiquimod for the methods provided herein can range from about 0.002 mg/kg (such as 0.005 mg/kg, 0.008 mg/kg, about 0.01 mg/kg, about 0.02 mg/kg, about 0.05 mg/kg, about 0.1 mg/kg, about 0.5 mg/kg, or about 1 mg/kg) to about 2 mg/kg in primates, such as about 0.9 mg/kg). Administration of Gardiquimod (and/or other imidazoquinoline compounds or derivative(s) that bind to and activate TLR7/8) to a subject at risk of an excitotoxic injury, ischemia and/or hypoxia, prior to such a cytotoxic insult protects against cell injury and death. In addition, administration of Gardiquimod (and/or other imidazoquinoline compounds or derivative(s) that bind to and activate TLR7/8) to a subject who has suffered an excitotoxic injury, ischemia and/or hypoxia, following such a cytotoxic insult treats cell injury and death. Thus, Gardiquimod (and related compounds or derivatives) can be used as an alternative to, or in combination with other TLR ligands, such as CpG oligonucleotides and poly I:C.

Yet another example of a TLR7/8 ligand is CL075. CL075 is a thiazoloquinolone derivative that stimulates TLR8 in human PBMC. It activates NF-κB and triggers preferentially the production of TNF-a and IL-12. In some embodiments, the structure of CL075 or CL075 derivative is:

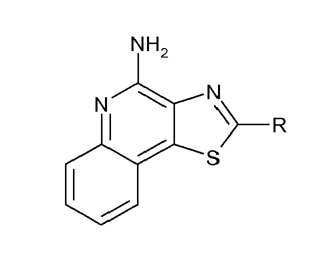

where R is an aliphatic group comprising 1-5 carbon atoms.

In other embodiments, the structure ofCL075 is:

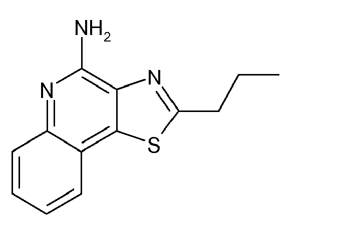

Exemplary therapeutic doses of CL075 for the methods provided herein can range from about 0.002 mg/kg (such as 0.005 mg/kg, 0.008 mg/kg, about 0.01 mg/kg, about 0.02 mg/kg, about 0.05 mg/kg, about 0.1 mg/kg, about 0.5 mg/kg, or about 1 mg/kg) to about 2 mg/kg in primates, such as about 0.9 mg/kg). Administration of CL075 to a subject at risk of an excitotoxic injury, ischemia and/or hypoxia, prior to such a cytotoxic insult protects against cell injury and death. In addition, administration of CL075 to a subject who has suffered an excitotoxic injury, ischemia and/or hypoxia, following such a cytotoxic insult treats cell injury and death. Thus, CL075 (and related compounds or derivatives) can be used as an alternative to, or in combination with other TLR ligands, such as poly I:C.

R848 (resiquimod) is an imidazoquinoline compound that activates immune cells via the TLR7/TLR8 MyD88-dependent signaling pathway. R848 triggers NF-κB activation in cells expressing murine TLR8 when combined with poly(dT). An exemplary structure of R848 is shown below.

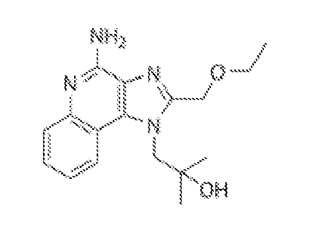

Exemplary therapeutic doses of R848 for the methods provided herein can range from about 0.002 mg/kg (such as 0.005 mg/kg, 0.008 mg/kg, about 0.01 mg/kg, about 0.02 mg/kg, about 0.05 mg/kg, about 0.1 mg/kg, about 0.5 mg/kg, or about 1 mg/kg) to about 2 mg/kg in primates, such as about 0.9 mg/kg). Administration of R848 to a subject at risk of an excitotoxic injury, ischemia and/or hypoxia, prior to such a cytotoxic insult protects against cell injury and death. In addition, administration of R848 to a subject who has suffered an excitotoxic injury, ischemia and/or hypoxia, following such a cytotoxic insult treats cell injury and death. Thus, R848 (and related compounds or derivatives) can be used as an alternative to, or in combination with other TLR ligands, such as poly I:C.

CL097 is a highly water-soluble derivative of the imidazoquinoline compound R848 (>20 mg/ml). Similarly to R848, CL097 is a TLR7 and TLR8 ligand. It induces the activation of NF-κB at 0.4 µM (0.1 µg/ml) in TLR7-transfected HEK293 cells and at 4 µM (1 µg/ml) in TLR8-transfected HEK293 cells. The structure of CL097 is shown below.

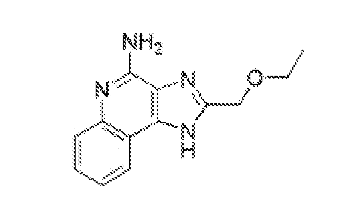

Exemplary therapeutic doses of CL097 for the methods provided herein can range from about 0.002 mg/kg (such as 0.005 mg/kg, 0.008 mg/kg, about 0.01 mg/kg, about 0.02 mg/kg, about 0.05 mg/kg, about 0.1 mg/kg, about 0.5 mg/kg, or about 1 mg/kg) to about 2 mg/kg in primates, such as about 0.9 mg/kg). Administration of CL097 to a subject at risk of an excitotoxic injury, ischemia and/or hypoxia, prior to such a cytotoxic insult protects against cell injury and death. In addition, administration of CL097 to a subject who has suffered an excitotoxic injury, ischemia and/or hypoxia, following such a cytotoxic insult treats cell injury and death. Thus, CL097 (and related compounds or derivatives) can be used as an alternative to, or in combination with other TLR ligands, such as poly I:C.

### TLR9 ligands

In one exemplary embodiment, a composition containing one or more TLR9 ligands (and in some examples with one or more other TLR ligands) is administered to a subject for the purpose of (1) treating one or more cells (or cell types, or tissues, or organs) following a or (2) preconditioning one or more cells (or cell types, or tissues, or organs) to protect against a subsequent, cytotoxic insult, such as an excitotoxic injury, ischemia or hypoxia.

TLR9 recognizes specific unmethylated CpG-ODN sequences that distinguish microbial DNA from mammalian DNA. In one exemplary embodiment, oligonucleotides containing an unmethylated CpG motif are administered to a subject for the purpose of treating or preconditioning one or more cells (or cell types, or tissues, or organs) to treat or protect against a cytotoxic insult, such as an excitotoxic injury, ischemia or hypoxia.

In the context of the present disclosure, CpG oligonucleotides (or CpG ODN) are oligonucleotides that contain at least one unmethylated cytosine-guanine dinucleotide sequence. The production and use of CpG oligonucleotides are known in the art, and described, for example, in US Patents No. 6,194,388 and 6,406,705. Without being bound by theory, all compositions and methods of producing them disclosed in US Patents No. 6,194,388 and 6,406,705 are incorporated herein by reference for all purposes.

Typically, a CpG ODN is between about 8 and 100 nucleotides in length. The CpG ODN is at least 10, or at least 12 nucleotides in length. Generally, a CpG ODN is no more than about 40 nucleotides in length. Although longer nucleotides can be employed, the cost of production increases with length with no significant benefit in terms of activity. Thus, while it is possible to use CpG ODN longer than 50 nucleotides, or 60 nucleotides, or even 70, or 80, or 90, or 100 nucleotides or more in length, there is little benefit in doing so. Frequently, the unmethylated CpG is flanked by complementary nucleotides, such that a palindromic sequence capable of hairpin formation (via base pairing interactions) around the CpG dinucleotide is included in the sequence of the CpG ODN. The inclusion of palindromic sequences flanking the unmethylated CpG dinucleotide can be desirable when using short oligonucleotides (e.g., 10 or 12 nucleotides in length).

CpG oligonucleotides bind to TLR9 on the surface of cells in a wide variety of tissues and/or organs (Nishimura and Naito, Biol. Pharm. Bull. 28:886-892, 2005). Binding of a CpG oligonucleotide to TLR9 initiates a signaling pathway mediated by MyD88 and p38-MAPK, which induces expression of NF-κB and IFNβ,among other genomic changes.

CpG oligonucleotides can be divided into at least three classes based on their structural and functional attributes. For example, A-Class CpG oligonucleotides (exemplified by SEQ ID NO: 2) stimulate dendritic cells to make large amounts of IFNa but have a weak effect on B cells. In contrast, B-Class CpG oligonucleotides (exemplified by SEQ ID NOS: 3 and 5) induce IFNa production to a lesser extent, but very strongly induce B cell activation and antibody production. C-Class oligonucleotides (exemplified by SEQ ID NO:4) combine the effects of A- and B-Class oligonucleotides by exhibiting strong B cell, IFNa stimulation, and natural killer cell activation. Any of these classes can be used to elicit a response in the context of the treatment or preconditioning methods disclosed herein. Specific exemplary CpG oligonucleotides are provided in SEQ ID NOS: 1-11. Another specific exemplary CpG oligonucleotide is 1018 ISS.

For use in the methods disclosed herein, oligonucleotides can be synthesized de novo using any of a number of procedures well known in the art. For example, the (β-cyanoethyl phosphoramidite method (Beaucage and Caruthers, Tet. Let. 22:1859, 1981); nucleoside H-phosphonate method (Garegg et al., Tet. Let. 27: 4051-4054, 1986; Froehler et al., Nucl. Acid. Res. 14: 5399-5407, 1986; Garegg et al., Tet. Let. 27: 4055-4058, 1986; Gafffney et al., Tet. Let. 29:2619-2622, 1988) can be used. These chemistries can be performed by a variety of commercially-available automated oligonucleotide synthesizers. Alternatively, oligonucleotides can be prepared from existing nucleic acid sequences (e.g., genomic or CDNA) using known techniques, such as those employing restriction enzymes, exonucleases or endonucleases.

For use *in vivo* oligonucleotides that are relatively resistant to degradation (such as, by endo- and exo- nucleases) can be used. An oligonucleotide that is relatively resistant to *in vivo* degradation is referred to as a "stabilized oligonucleotide." Oligonucleotide stabilization can be accomplished via phosphate backbone modifications. For example, an ODN can be rendered nuclease resistant by phosphorothioate modification (that is, at least one of the phosphate oxygens is replaced by sulfur) of one or more internucleotide linkages. In some cases, the terminal internucleotide linkages are phosphorothioate modified. Procedures for synthesizing phosphorothioate modified CpG oligonucleotides are disclosed, e.g., in U.S. Patents Nos. 5,663,153 and 5,723,335, the disclosures of which are incorporated herein by reference.

The pharmacokinetics of phosphorothioate ODN show that they have a systemic half-life of forty-eight hours in rodents and are useful for *in vivo* applications (Agrawal et al. Proc. Natl. Acad. Sci. USA 88:7595-7599, 1991). Phosphorothioates can be synthesized using automated techniques employing either phosphoramidate or H phosphonate chemistries.

Other stabilized oligonucleotides include: nonionic DNA analogs, such as alkyl- and aryl-phosphonates (in which the charged phosphonate oxygen is replaced by an alkyl or aryl group), phosphodiester and alkylphosphotriesters, in which the charged oxygen moiety is alkylated. Oligonucleotides which contain a diol, such as tetraethyleneglycol or hexaethyleneglycol, at either or both termini have also been shown to be substantially resistant to nuclease degradation. Aryl- and alkylphosphonates can be made (for example, as described in U.S. Patent No. 4,469,863); and alkylphosphotriesters (in which the charged oxygen moiety is alkylated as described in U.S. Patent No. 5,023,243 and European Patent No. 092,574) can be prepared by automated solid phase synthesis using commercially available reagents. Methods for making other DNA backbone modifications and substitutions have been described (Uhlmann and Peyman, Chem. Rev. 90:543-584, 1990; Goodchild, Bioconjugate Chem. 1:165-187, 1990).

For administration *in vivo,* CpG oligonucleotides can be associated with a molecule that enhances binding to target cell surfaces and/or increased cellular uptake by target cells to form an "oligonucleotide delivery complex." Oligonucleotides can be ionically, or covalently associated with appropriate molecules using techniques which are well known in the art. A variety of coupling or crosslinking agents can be used, including protein A, carbodiimide, and N-succinimidyl-3-(2-pyridyldithio) propionate (SPDP). Alternatively, oligonucleotides can be encapsulated in liposomes or virosomes using well-known techniques to facilitate delivery.

Oligonucleotides containing at least one unmethylated CpG dinucleotide can be administered to a subject *in vivo* in the methods provided herein, for example to treat or prevent the adverse effects of excitotoxic injury, ischemia and/or hypoxia. CpG oligonucleotides are systemically administered to a subject how has suffered from or is at risk for one or more of excitotoxic, ischemic and/or hypoxic injury. An effective amount of an appropriate oligonucleotide (alone or formulated as an oligonucleotide delivery complex) can be administered to a subject by any mode allowing the oligonucleotide to bind to appropriate receptors on the surface of target cells (e.g., TLR9). Formulation and dosages of compositions containing CpG oligonucleotides are discussed in detail below.

### Identification of other TLR ligands

Additional TLR ligands (or other agonists that specifically bind to and activate a particular TLR) can be identified using a reporter system in which binding and activation of a selected TLR and induction of NF-KB is detected using an NF-κB responsive reporter construct. Cell lines, such as HEK293, stably transfected with the components necessary for signaling via a selected TLR are transfected with an NFκB inducible reporter plasmid, pNiFty2-SEAP (InvivoGen, San Diego). This plasmid contains an engineered promoter that combines five NFκB sites with the proximal ELAM (endothelial cell-leukocyte adhesion molecule) promoter upstream of a reporter gene encoding secreted alkaline phosphatase (SEAP). SEAP is extremely heat stable and can be detected spectrophotometrically, either colorimetrically or by detecting a luminescent product, e.g., using a PHOSPHA-LIGHT™ chemiluminescence kit (Applied Biosystems, BP3000). In this assay, the substrate CSPD [3-(4-methoxyspiro [1,2-dioxetane-3,2(5'-chloro)-tricyclo(3.3.1.13,7)decane]-4-yl)phenyl phosphate] is dephosphorylated by SEAP, and the resulting unstable dioxetane anion decomposes and emits light at a wavelength of 477 nm. The light signal is quantified (for example using a microplate luminometer) and is linear up to five orders of magnitude and proportional to the concentration of SEAP. The extent of TLR activation can be quantified by collecting supernatant and determining the concentration of SEAP via this assay.

Cell lines expressing mouse and human TLRs 1-10 are commercially available (e.g., from InvivoGen) or can be produced by those of skill in the art using routine molecular biology procedures using publicly available TLR sequences, for example as described in Sambrook et al., Molecular Cloning: a Laboratory Manual, Cold Spring Harbor Press, 1989.

In brief, the transfected cell line expressing the selected TLR and the parental HEK293 cells each carrying the NF-κB reporter construct are stimulated (for between 12 and 24 hours, e.g., for approximately 18 hours or overnight) with varying doses of a test agent. Typically, each test agent is tested at multiple doses to determine a dose/response curve. Following the incubation, supernatant is collected and NF-κB activity is measured using an alkaline phosphatase assay.

Following detection of an agent that binds to and activates the selected TLR and induces NF-κB, the preconditioning capacity of the agent can be confirmed using an *in vitro* model of stroke. Primary mouse cortical neuronal cultures are pretreated with media or the identified test agent at a suitable dose determined from the dose/response curve. Approximately 24 hours later, the growth medium is replaced with PBS containing 0.5 mM CaCl₂ and 5 mM MgCl₂, pH 7.4. The neuronal cultures are placed in an anaerobic chamber containing an atmosphere of 85% N₂, 5% H₂, 10% CO₂ that is maintained at 35°C (oxygen-glucose deprivation-treatment, "OGD"). Following OGD-treatment (3 hours), the PBS is replaced with Minimum Essential Medium (MEM) and the cultures are returned to normoxic conditions.

Similarly, following detection of an agent that binds to and activates the selected TLR and induces NF-κB, the treatment capacity of the agent can be confirmed using an *in vitro* model of stroke. Primary mouse cortical neuronal cultures in PBS containing 0.5 mM CaCl₂ and 5 mM MgCl₂, pH 7.4 are are placed in an anaerobic chamber containing an atmosphere of 85% N₂, 5% H₂, 10% CO₂ that is maintained at 35°C (oxygen-glucose deprivation-treatment, "OGD"). Following OGD-treatment (3 hours), the PBS is replaced with Minimum Essential Medium (MEM) and the cultures are returned to normoxic conditions. During this time (for example 24 hours following OGD), the identified test agent can be added at a suitable dose determined from the dose/response curve to determine the ability of the agent to treat or rescue the cells from injury due to the OGD conditions.

Percent cell death is then determined. For example, background cell death can be determined using medium and the test agent alone without OGD, to determine the effect of each compound on cell viability. LPS (1 µg/ml) preconditioning of a TLR4 transfected cell line can be used as a positive control for neuronal protection and poly I:C (1 to 100 µg/ml) can be used as a positive control for neuronal treatment. Cell death is assessed approximately 24 hours following OGD or following treatment with the test agent using, for example, the fluorescent exclusion dye propidium iodide. The percent cell death is quantified, and differences between means (% cell death) in the cells contacted with the test agent and controls are compared for significance, e.g., using between groups factorial ANOVA grouped on treatment (media vs. test agent) and hypoxic status (no OGD vs. OGD).

Agents that exhibit a significant protective effect, such as at least about a 20% decrease in cell death, as compared to cultures that were not exposed to the agent *(e.g.,* medium alone), are suitable as preconditioning agents to protect against excitotoxic injury and/or hypoxia. Similarly, agents that exhibit a significant treatment effect, such as at least about a 20% decrease in cell death, as compared to cultures that were not exposed to the agent (e.g., medium alone), are suitable as treatment agents to protect against excitotoxic injury and/or hypoxia. The methods described above can be used to screen libraries of compounds, such as Mixture Based Positional Scanning Libraries, for preconditioning agents. A Mixture-Based Positional Scanning Library is designed to provide information on the activity of collections of systematically arranged compounds numbering in the thousands to millions. The positional scanning technology has been used successfully to identify novel enzyme inhibitors, receptor agonists and antagonists, antimicrobial, antifungal, and antiviral compounds (Houghten et al., J. Med. Chem. 42:3743-3778, 1999; Pinilla et al., Nat. Med. 9:118-122, 2003). In addition, this technology has been independently validated by a number of research groups. Publications from more than 100 separate studies carried out by approximately 50 research laboratories (Houghten et al., J. Med. Chem. 42:3743-3778, 1999) reflect the broad utility of screening systematically arranged collections of compounds, such as positional scanning libraries.

Each positional scanning library is designed around a core pharmacophore. Traditionally, core pharmacophores in positional scanning libraries are chosen based on the following criteria: the core structure can be produced under straightforward and inexpensive synthetic conditions; the core structure can have numerous incorporated functional diversity elements; and the core structure is known or purported to be of biological importance. Each positional scanning sub-library contains positions that enable structural variations around the central core. Screening data from a library provides extensive structure-activity relationship information and enables identification of active individual compounds. Thus the individual structural components and their representative contributions to total biological activity within the positional scanning library are revealed. Mixture-based small molecule positional scanning combinatorial libraries (Mixture Sciences, Inc.) can be screened to identify agents that activate a selected TLR. Typically, human TLR are utilized to identify agents with optimal activity characteristics for human receptors.

Thus, in an exemplary screening protocol, a transfected cell line expressing the selected TLR is first contacted with pools of libraries to identify libraries with active constituents. Library mixtures and library pools are formulated at 1 mg/ml in 10% dimethyl formamide (DMF). Cell lines are tested for toxicity to DMF at concentrations of 1%or less. Library pools are applied at the maximum practical concentration, determined by the cell line's tolerance to DMF.

Appropriate concentrations of DMF alone, as well as LPS or poly I:C in DMF controls, are run in duplicate in each 96 well assay plate. Following the incubation period, supernatant is collected for an alkaline phosphatase assay. Active libraries are screened subsequently as individual mixtures to identify the most active functional groups on each library scaffold. The compounds predicted to be most active are synthesized and tested as individual compounds. Ligands that show both TLR binding (and activation) and protective or treatment properties in an *in vitro* model can be further evaluated for their protective characteristics in an in *vivo* model of stroke.

### Pharmaceutical Compositions and Methods

The TLR ligand-containing compositions (medicaments) disclosed herein can be administered to a subject to protect against excitotoxic injury, ischemia and/or hypoxia. Accordingly, the compositions are administered to a subject at risk of an excitotoxic, ischemic or hypoxic event to prevent or reduce the deleterious effects of such an event or occurrence. Administration of the composition is not necessarily deemed to alter the likelihood of occurrence of any cytotoxic insult, rather administration of the preconditioning composition alters or modifies the outcome following the occurrence of such an event by inducing cellular changes (e.g., in the genomic program) that reduce, prevent or ameliorate the effects of the excitotoxic, ischemic and/or hypoxic event.

In other examples, the TLR ligand-containing compositions (medicaments) disclosed herein can be administered to a subject following an excitotoxic injury, ischemia and/or hypoxia. Accordingly, the compositions are administered to a subject following an excitotoxic, ischemic or hypoxic event to treat the deleterious effects of such an event or occurrence.

The therapeutic compositions include at least one agent that binds to a TLR, such as a TLR ligand or angonist. Thus, as disclosed herein, the pharmaceutical compositions can include one or more agent that is a ligand of a TLR. The ligand is selected to be appropriate for the subject receiving the composition. For example, when administering a therapeutic agent to a human subject, the agent is selected to be a ligand that binds to and activates a human TLR. Similarly, if the subject to be treated is a non-human animal, the agent is selected to bind to and activate a TLR of that species of animal. It should be noted that some TLR ligands bind to human as well as animal TLRs, whereas other ligands bind TLRs of some but not other species. One of skill in the art can confirm appropriate TLR-binding of a selected ligand empirically without undue experimentation. In some cases, the composition includes a single TLR ligand; in other instances, the composition includes more than one TLR ligand. Where a composition includes more than one TLR ligand, the composition can include multiple agents that bind to and activate a single TLR (optionally with different signaling results) or that bind to and activate different TLRs.

The quantity of the TLR ligand or agonist (such as a TLR3, TLR7, TLR7/8, or TLR9 ligand or agonist) included in the pharmaceutical composition is an amount determined to provide a treatment or preconditioning effect. In one example, the dose administered to a human subject is the same biological equivalent (EC₅₀) observed in other primates. The disclosed compositions and methods can be used to increase or decrease immune cell numbers or activation of an immune cell in a subject with or at risk of an ischemic event. In one embodiment, a method includes administering to a subject an amount of a TLR ligand sufficient to increase or decrease immune cell numbers or activation state of the immune cells in the subject. In another embodiment, a method includes administering to a subject an amount of TLR ligand sufficient to increase immune cell numbers or activation of the immune cell in the subject. Non-limiting exemplary immune cells include T cells, monocytes, macrophages, and dendritic cells (DC). Additional non-limiting exemplary immune cells include B cells, NK cells, NKT cells and granulocytes.

In a specific example, when administered to a subject (such as a human subject) in one or more doses, a composition can include an amount of a TLR ligand sufficient to provide at least about 0.005 mg of the TLR ligand per kg body weight of the subject (0.005 mg/kg). Thus, exemplary compositions include an amount of a TLR ligand (such as a CpG oligonucleotide, Gardiquimod, imiquimod, CL075, R848, or poly I:C) from about 0.008 mg/kg (for example, about 0.01 mg/kg, or about 0.02 mg/kg, or about 0.025 mg/kg, or about 0.05 mg/kg) to about 5 mg/kg (for example, about 0.08 mg/kg, about 0.09 mg/kg, about 0.10 mg/kg, about 0.12 mg/kg, about 0.15 mg/kg, about 0.5 mg/kg, about 1 mg/kg or about 2 mg/kg). Thus, for administration to an adult human, a the composition can be formulated to include at least about 0.1 mg (100 µg) of a TLR ligand, to about 250 mg of the TLR ligand, in a single dose. One skilled in the art will recognize that suitable dose ranges and dosage can be determined by one of skill in the art for any TLR binding agent with a therapeutic effect.

Methods for formulating and delivering CpG oligonucleotides are provided in US Patents No: 6,194,388 and 6,406,705. The methods of formulating and administering CpG oligonucleotides disclosed therein are incorporated herein by reference.

The TLR ligand-containing composition typically includes one or more pharmaceutically acceptable constituents, such as a pharmaceutically acceptable carrier and/or pharmaceutically acceptable diluent. Typically, preparation of a therapeutic composition entails preparing a pharmaceutical composition that is essentially free of pyrogens, as well as any other impurities that could be harmful to humans or animals. Typically, the pharmaceutical composition contains appropriate salts and buffers to render the components of the composition stable and facilitate administration to a subject. Such components can be supplied in lyophilized form, or can be included in a diluent used for reconstitution of a lyophilized form into a liquid form suitable for administration. Alternatively, where the medicament is prepared for administration in a solid state (e.g., as a powder or pellet), a suitable solid carrier is included in the formulation.

Aqueous compositions typically include an effective amount of the TLR ligand dispersed (for example, dissolved or suspended) in a pharmaceutically acceptable diluent or aqueous medium. Pharmaceutically acceptable molecular entities and compositions generally do not produce an adverse, allergic or other undesirable reaction when administered to a human or animal subject. As used herein, pharmaceutically acceptable carriers include any and all solvents, dispersion media, coatings, isotonic and absorption delaying agents, and the like. Optionally, a pharmaceutically acceptable carrier or diluent can include an antibacterial, antifungal or other preservative. The use of such media and agents for pharmaceutically active substances is well known in the art. Except insofar as any conventional media or agent is incompatible with production of a treatment or preconditioning response, its use in the disclosed compositions is contemplated. In some cases (for example, when liquid formulations are deemed desirable, or when the agent is reconstituted for multiple doses in a single receptacle), these preparations contain a preservative to prevent or inhibit the growth of microorganisms.

Pharmaceutically acceptable carriers, excipients and diluents are known to those of ordinary skill in the described, *e.g.,* in Remington's Pharmaceutical Sciences, by E. W. Martin, Mack Publishing Co., Easton, PA, 15th Edition (1975), describes compositions and formulations suitable for pharmaceutical delivery of TLR ligands.

In general, the nature of the carrier will depend on the particular mode of administration being employed. For instance, parenteral formulations usually include injectable fluids that include pharmaceutically and physiologically acceptable fluids such as water, physiological saline, balanced salt solutions, aqueous dextrose, glycerol or the like as a vehicle. For solid compositions (e.g., powder, pill, tablet, or capsule forms), conventional non-toxic solid carriers can include, for example, pharmaceutical grades of mannitol, lactose, starch, or magnesium stearate. In addition to biologically neutral carriers, pharmaceutical compositions to be administered can contain minor amounts of non-toxic auxiliary substances, such as wetting or emulsifying agents, preservatives, and pH buffering agents and the like, for example, sodium acetate or sorbitan monolaurate.

For example, the pharmaceutical compositions can include one or more of a stabilizing detergent, a micelle-forming agent, and an oil. Suitable stabilizing detergents, micelle-forming agents, and oils are detailed in U.S. Patents No. 5,585,103; 5,709,860; 5,270,202; and 5,695,770. A stabilizing detergent is any detergent that allows the components of the emulsion to remain as a stable emulsion. Such detergents include polysorbate, 80 (TWEEN) (Sorbitan-mono-9-octadecenoate-poly(oxy-1,2-ethanediyl; manufactured by ICI Americas, Wilmington, DE), TWEEN 40™, TWEEN 20™, TWEEN 60™, Zwittergent™ 3-12, TEEPOL HB7™, and SPAN 85™. These detergents are typically provided in an amount of approximately 0.05 to 0.5%, such as at about 0.2%.

A micelle forming agent is an agent which is able to stabilize the emulsion formed with the other components such that a micelle-like structure is formed. Such agents generally cause some irritation at the site of injection in order to recruit macrophages to enhance the cellular response. Examples of such agents include polymer surfactants described by, e.g., Schmolka, J. Am. Oil. Chem. Soc. 54:110, 1977, and Hunter et al., J. Immunol 129:1244, 1981, and such agents as PLURONIC™ L62LF, L101, and L64, PEG1000, and TETRONIC™ 1501, 150R1, 701, 901, 1301, and 130R1. The chemical structures of such agents are well known in the art. In one embodiment, the agent has a hydrophile-lipophile balance (HLB) of between 0 and 2, as defined by Hunter and Bennett (J. Immun. 133:3167, 1984). The agent can be provided in an effective amount, for example between 0.5 and 10%, or in an amount between 1.25 and 5%.

The oil included in the composition is chosen to promote the retention of the TLR ligand in oil-in-water emulsion, and can have a melting temperature of less than 65°C, such that emulsion is formed either at room temperature, or once the temperature of the emulsion is adjusted to room temperature. Examples of such oils include squalene, squalane, EICOSANE™, tetratetracontane, glycerol, and peanut oil or other vegetable oils. In one specific, non-limiting example, the oil is provided in an amount between 1 and 10%, or between 2.5 and 5%. The oil should be both biodegradable and biocompatible so that the subject can break down the oil over time, and so that no adverse affects, such as granulomas, are evident upon use of the oil.

The pharmaceutical compositions (medicaments) can be prepared for use in the regimens provided herein and administered to human or non-human subjects to elicit a treatment or protective response against an excitotoxic, ischemic or hypoxic event. For example, the compositions described herein can be administered to a human (or non-human) subject to elicit a treatment or protective response against stroke or other ischemic events.

A pharmaceutical composition containing one or more TLR ligands (for example, containing a CpG oligonucleotide, Gardiquimod, imiquimod, R848, CL075, or poly I:C) can be administered by any means known to one of skill in the art, such as inhalation, injection, transdermal or oral routes, for example nasal, intravenous, intramuscular, or subcutaneous injection, so long as the route of administration results in systemic (as opposed to localized) distribution of the therapeutic agent. In one embodiment, administration is intranasal.

As an alternative to liquid formulations, the TLR ligand-containing composition can be administered in solid form, e.g., as a powder, pellet or tablet. For example, the preconditioning agent can be administered as a powder using a transdermal needleless injection device, such as the helium-powered POWDERJECT® injection device. This apparatus uses pressurized helium gas to propel a powder formulation of a TLR ligand-containing composition, e.g., containing a CpG oligonucleotide or poly I:C, at high speed so that the particles perforate the stratum corneum and contact cells in the epidermis.

Polymers can be also used for controlled release. Various degradable and nondegradable polymeric matrices for use in controlled drug delivery are known in the art (Langer, Accounts Chem. Res. 26:537, 1993). For example, the block copolymer polaxamer can exist as a viscous yet mobile liquid at low temperatures but forms a semisolid gel at body temperature (Johnston et al., Pharm. Res. 9:425, 1992; and Pec, J. Parent. Sci. Tech. 44(2):58, 1990). Alternatively, hydroxyapatite has been used as a microcarrier for controlled release (Ijntema et al., Int. J. Pharm. 112:215, 1994). In yet another aspect, liposomes are used for controlled release as well as drug targeting of the lipid-capsulated drug (Betageri et al., Liposome Drug Delivery Systems, Technomic Publishing Co., Inc., Lancaster, PA, 1993). Numerous additional systems for controlled delivery of therapeutic compositions are known (e.g., U.S. Patents No. 5,055,303; 5,188,837; 4,235,871; 4,501,728; 4,837,028; 4,957,735; 5,019,369; 5,055,303; 5,514,670; 5,413,797; 5,268,164; 5,004,697; 4,902,505; 5,506,206; 5,271,961; 5,254,342; and 5,534,496).

In one example, the disclosed compositions are administered following to the occurrence of an excitotoxic, ischemic or hypoxic event. Generally, the composition is administered at least 30 minutes following the event, such as at least 1 hour, at least 2 hours, at least 6 hours, at least 24 hours, or at least 48 hours following the event. The treatment effects of a single administration of a TLR ligand, such as a poly I:C may last for greater than one week (e.g., up to about 10 days, or more). In some examples, multiple administrations are given following the event (such as for weeks, months or years following the event). For example, in the case of an ongoing event, such as in the case of Alzheimer's disease, multiple administrations are given, for example on a predetermined schedule, such as at weekly intervals. Alternatively, the composition can be formulated and administered on a continuous basis, for example using a pump (or other intravenous or intrathecal) infusion method. The individual treatment regimen can be customized to the particular event or activity, such that the therapeutic effects of the dose of the TLR ligand are optimized under the particular circumstances for the particular subject.

In one example, the disclosed compositions are administered prior to the occurrence of an excitotoxic, ischemic or hypoxic event (or prior to an increase in the likelihood of such an event). Generally, the composition is administered at least 10 hours prior to the event or activity, in order to fully realize the preconditioning effect of administration. Usually, the composition is administered at least 24 hours before the event or activity. The protective effects of a single administration of a preconditioning agent, such as a TLR ligand, last for greater than one week (e.g., up to about 10 days, or more). Thus, in the case of an isolated event, that is, an event that is not predicted to be a recurring event, such as a surgical operation, the composition is given prior to the commencement of the event, such as about 10 hours, or about 12 hours, or about 24 hours prior to the event or activity, and can be given up to about 1 week prior to the event, and in some cases up to about 10 days or more prior to the event. In the case of a recurrent event, such as repeated engagement in a contact sport, multiple administrations are given, the ultimate dose (that is, the most recent dose prior to the event) being given prior (such as, at least 10 hours, or up to about 1 week, prior) to the event or activity. Similarly, in the case of an ongoing event, such as in the case of Alzheimer's disease, multiple administrations are given, for example on a predetermined schedule, such as at weekly intervals. Alternatively, the composition can be formulated and administered on a continuous basis, for example using a pump (or other intravenous or intrathecal) infusion method. The individual treatment regimen can be customized to the particular event or activity, such that the protective effects of the preconditioning dose of the agent (such as a CpG oligonucleotide, Gardiquimod, imiquimod, or CL075) are optimized under the particular circumstances for the particular subject.

### EXAMPLES

### Example 1: Preconditioning with CpG oligonucleotide or imiquimod confers neuroprotection in an in vitro ischemia model

This example provides an exemplary *in vitro* model of neuronal ischemia, and demonstrates that preconditioning with CpG oligonucleotides protects against hypoxia.

***In vitro* mouse neuronal cultures:** Cortical neuronal cultures were prepared as described Jin et al. (Neurochem. Res. 27:1105-1112, 2002) from E-16 mouse pups (C57B1/6, Jackson labs). Briefly, cortices were dissected and separated from meninges, olfactory bulbs, basal ganglia and hippocampi, and the cortices digested in 0.05 % trypsin-EDTA for 15 min at 37°C. Cells were triturated and single cell suspension was plated at density of 5 x 10⁵ cells/ml. Cells were cultured in Neurobasal A medium (Invitrogen, Carlsbad) containing 2% B27, 2mM Glutamate. Neuronal enrichment was determined by staining for neurons, microglia and astrocytes with cell specific markers.

***In vitro* neuronal ischemia model:** Neuronal cultures were treated with varying doses of an exemplary CpG oligonucleotide (SEQ ID NO: 1) in Neurobasal-A media supplemented with 1 % Glutamax 24 hours prior to 3 hours oxygen-glucose-deprivation (OGD) treatment. OGD was performed by replacing medium with PBS containing 0.5 mM CaCl₂ and 5 mM MgCl₂, pH 7.4, and then placing the neuronal cultures in an anaerobic chamber (Forma Scientific) containing an atmosphere of 85% N₂, 5% H₂, 10% CO₂ maintained at 35°C. Following OGD-treatment (3 *hours),* PBS was replaced with Minimum Essential Medium (MEM) and the cells returned to normoxic conditions. Percent neuronal cell death was determined by propidium iodide staining in two different fields of view in duplicate, compared to total DAPI staining in identical fields.

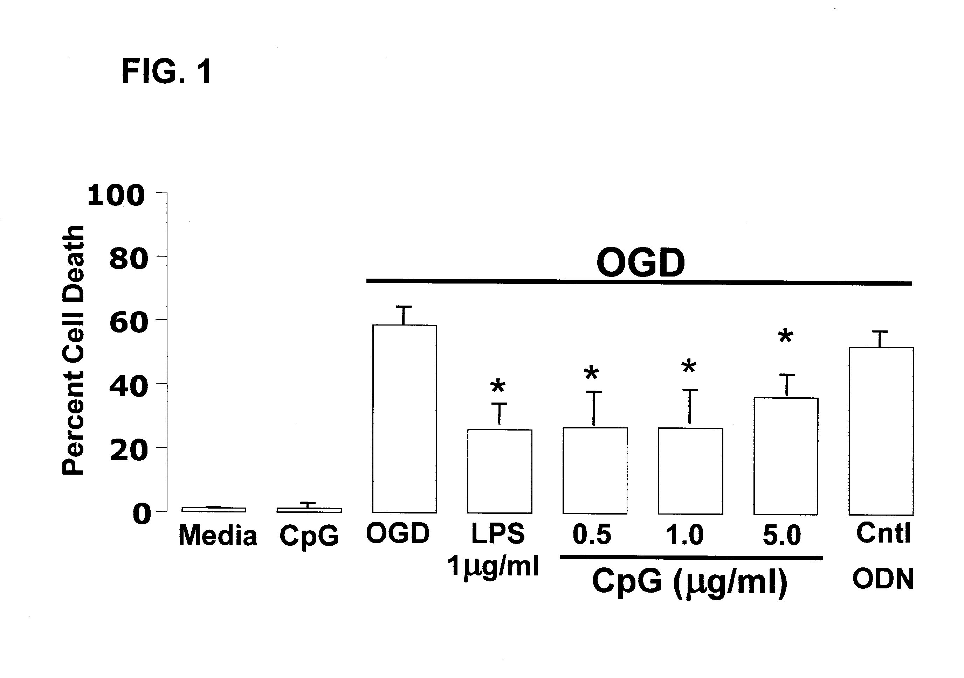

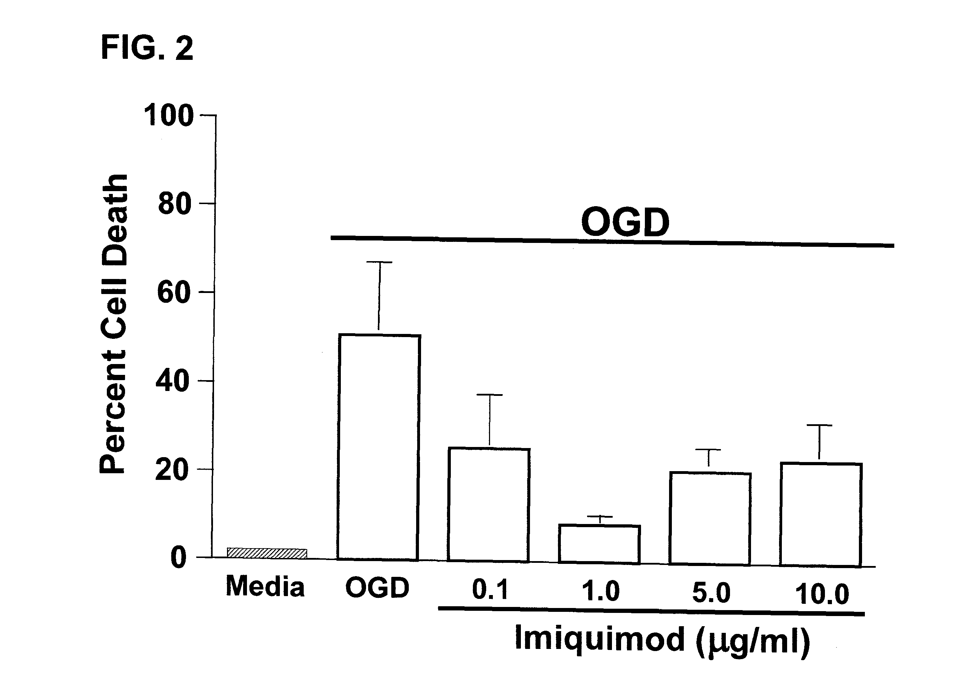

As shown in FIG. 1, the exemplary oligodeoxynucleotide containing an unmethylated CpG motif (SEQ ID NO: 1) confers neuroprotection against oxygen-glucose deprivation in mouse neuronal cultures. Similar results were obtained with imiquimod, as shown in FIG. 2.

### Example 2: NF-κB induction by CpG oligonucleotides in TLR9-expressing cells

This example provides an exemplary reporter system for detecting binding and activation of a TLR. Using this model, results are provided that demonstrate that CpG oligonucleotides that bind to TLR9 activate signaling via the receptor and induce NF-κB activity.

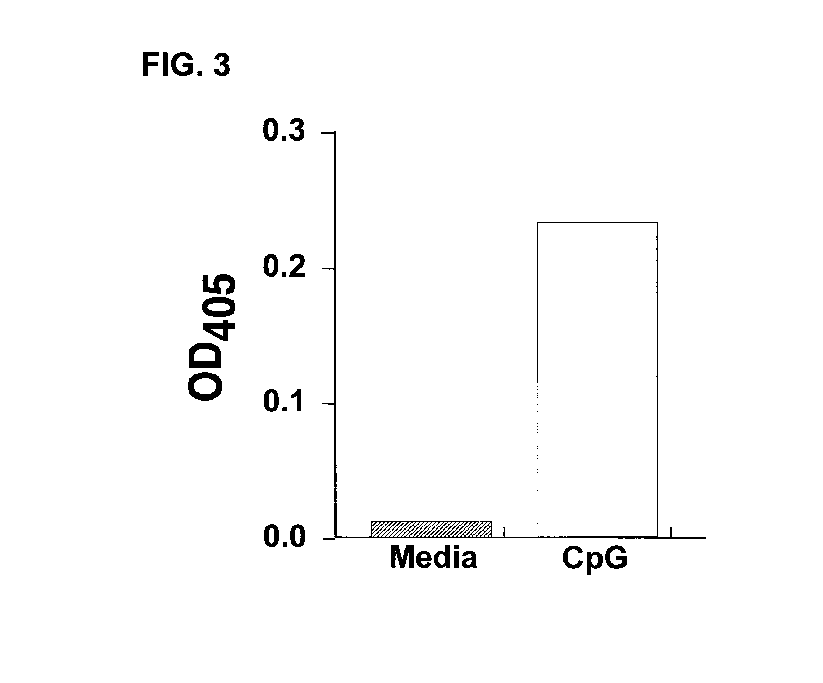

Human embryonic kidney cell line HEK293 was transfected with an expressible nucleic acid encoding human TLR9 and with an NFκB reporter construct (InvivoGen). The dual transfected cells were incubated with a 5 µM CpG oligonucleotide (SEQ ID NO: 1) for 18 hours. Following stimulation with the CpG oligonucleotide (SEQ ID NO: 1), the NFκB inducible reporter plasmid (pNiFty2-SEAP; InvivoGen) produced alkaline phosphatase, which was measured calorimetrically following substrate hydrolysis (FIG. 3).

### Example 3: Preconditioning with an exemplary CpG oligonucleotide in an in vivo Ischemic/Reperfusion Model

This example demonstrates that prophylactic administration of a composition containing a CpG oligonucleotide is neuroprotective in a mouse model of stroke.

**Intraperitoneal Delivery.** Preconditioning agent (20 µg CpG oligonucleotide (SEQ ID NO: 1) in artificial cerebrospinal fluid (aCSF) or aCSF alone (control) was administered intraperitoneally to subject mice at designated timepoints prior to middle cerebral artery occlusion (MCAO) as described below.

**Ischemic/Reperfusion Model.** Following administration of a preconditioning agent or control composition, adult (~3 months old) male C57BL/6 mice were subjected to 45 min MCAO according to the monofilament suture method previously described in detail (Hill et al., Brain Res. 820:45-54, 1999). Mice were anesthetized by halothane inhalation (4%/L O₂) and maintained with 1.5%/L O₂. The middle cerebral artery was blocked by a silicone-coated 8-0 monofilament nylon surgical suture that was threaded through the external carotid to the internal carotid and finally blocks the bifurcation into the MCA and anterior cerebral artery. The filament was maintained intraluminally for 45 min and then removed, thereby restoring blood flow. Cerebral blood flow (CBF) was monitored throughout the surgery by laser Doppler flowmetry (Periflow 5000; Perimed, Sweden). During and 2 hours following surgery, body temperatures was kept constant at 37°C with a heating pad controlled by a thermostat. Body weights were monitored prior to and following MCAO. Neurological testing is performed prior to sacrifice as published previously (Hill et al., Brain Res. 820:45-54, 1999).

**Motor Functions Tests.** Following pretreatment and/or ischemia/reperfusion, damage due to stroke is assessed using several behavioral indices of neurological function. The corner test correlates with infarct volume and reveals post-infarct recovery (Wang et al., Stroke 35:1732-1737, 2004). The test measures the extent to which the mouse favors (turns toward) the ipsilateral side after moving into a confining corner. The assessment is conducted as previously described (Zhang et al., J. Neurosci Methods 117:207-214, 2002). Each mouse is tested 10 times per session. The footfault test, which assesses forelimb dysfunction, does not predict infarct size but reflects recovery after MCAO (Wang et al., Stroke 35:1732-1737, 2004) and neuroprotection (Gibson and Murphy, J. Cereb. Blood Flow Metab. 24:805-813, 2004). Mice are assessed for missteps while walking on an elevated wire grid. Data (footfaults) are expressed as a fraction of the total number of steps taken (Zhang et al., J. Neurosci Methods 117:207-214, 2002). The tactile adhesive removal test, which probes somatosensory function, is conducted as described (Lindner et al., J. Neurosci. 23:10913-10922, 2003). Briefly, small adhesive paper spots are attached to the distal portion of each forelimb and the time required to remove the paper with the mouth is determined (3 trials per sessions separated by 1 minute each). Additionally, mice are evaluated for neurological symptoms using other standard indicia of mouse behavior.

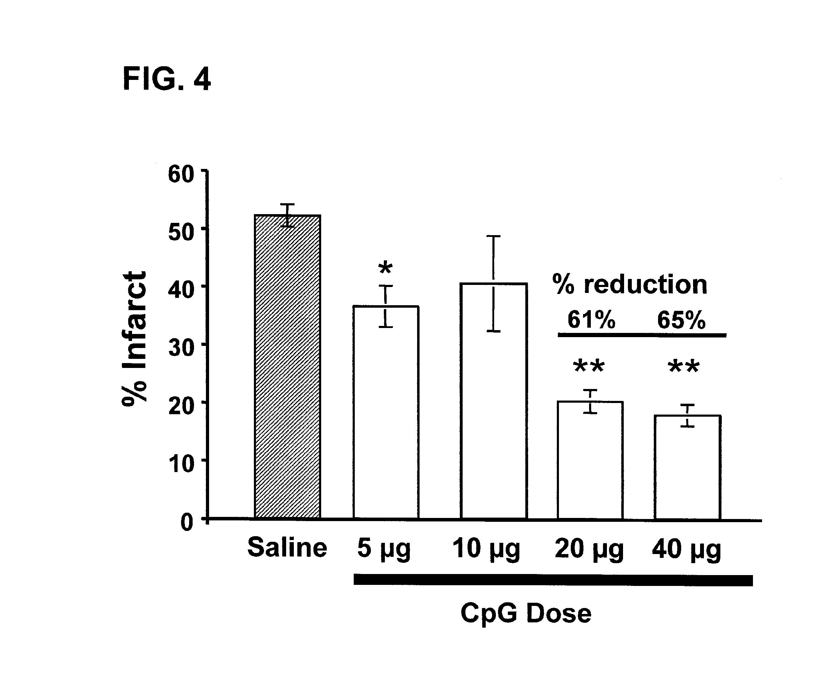

**Infarct calculations.** Following MCAO, mice were anesthetized with isoflurane and perfused with heparinized buffer to remove cells in the blood (Ford et al., J. Immunol. 154:4309-4321, 1995). Perfused brains were placed on a tissue slicer and sectioned into 1 mm thick coronal slices. To visualize the region of infarction, sections were stained with 1.5%, 2,3,4, triphenyltetrazolium chloride (TTC) in 0.9% phosphate buffered saline (Bederson et al., Stroke 17:1304-1308, 1986). Infarct size determination was performed using a computerized image analysis system according to principles described previously to eliminate edema measurement artifacts (Swanson et al., J. Cereb. Blood Flow Metab. 10:290-293, 1990). As shown graphically in FIG. 4, percent infarct was significantly decreased in mice treated with a preconditioning dose of an exemplary CpG oligonucleotide.

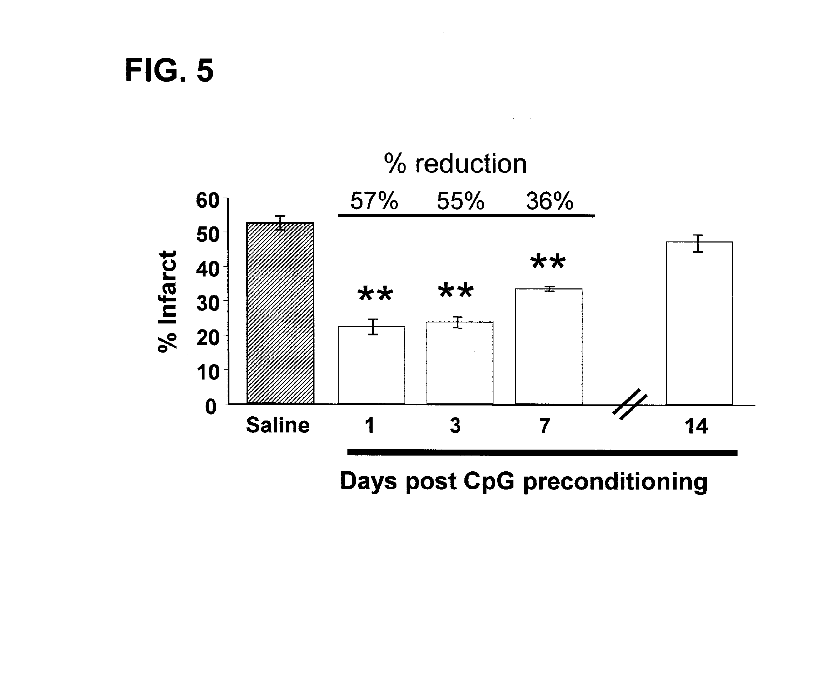

**Time course of preconditioning.** A time course for the preconditioning effects of CpG oligonucleotide administration was determined by administering 20 µg CpG oligonucleotide at intervals prior to MCAO, and evaluating percent infarct after MCAO as described above. Although peak preconditioning was observed following administration between 72 and 24 hours prior to MCAO, significant preconditioning was observed when a preconditioning dose of CpG oligonucleotide was administered up to a week prior to experimentally induced ischemia (FIG. 5).

### Example 4: Preconditioning with Imiquimod in an in vivo Ischemic/Reperfusion Model

This example demonstrates that prophylactic administration of imiquimod, a TLR7/8 binding agent, is neuroprotective in a mouse model of stroke.

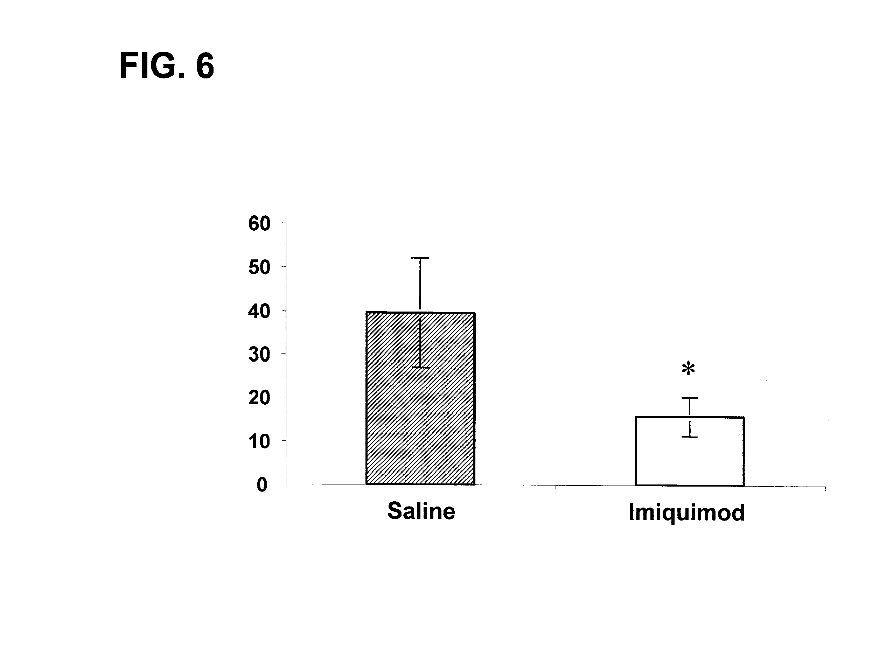

Imiquimod (20 µg) in artificial cerebrospinal fluid (aCSF) or aCSF alone (control) was administered intraperitoneally to subject mice 72 hours prior to 40 minute MCAO performed as described above. Brains were analyzed for infarct size as indicated in Example 3. FIG. 6 graphically illustrates that preconditioning with imiquimod protects against cell death in this *in vivo* model of stroke.

### Example 5: Preconditioning with Gardiquimod confers neuroprotection in an in vitro ischemia model

This example provides an exemplary *in vitro* model of neuronal ischemia, and demonstrates that preconditioning with Gardiquimod (a TLR7 agonist) protects against hypoxia.

***In vitro* mouse neuronal cultures:** Cortical neuronal cultures were prepared as described in Example 1.

***In vitro* neuronal ischemia model:** Neuronal cultures were treated with varying doses (0.1 µg/ml and 1 µg/ml) of Gardiquimod in Neurobasal-A media supplemented with 1 % Glutamax 24 hours prior to 3 hours oxygen-glucose-deprivation (OGD) treatment. OGD was performed and cells analyzed as described in Example 1.

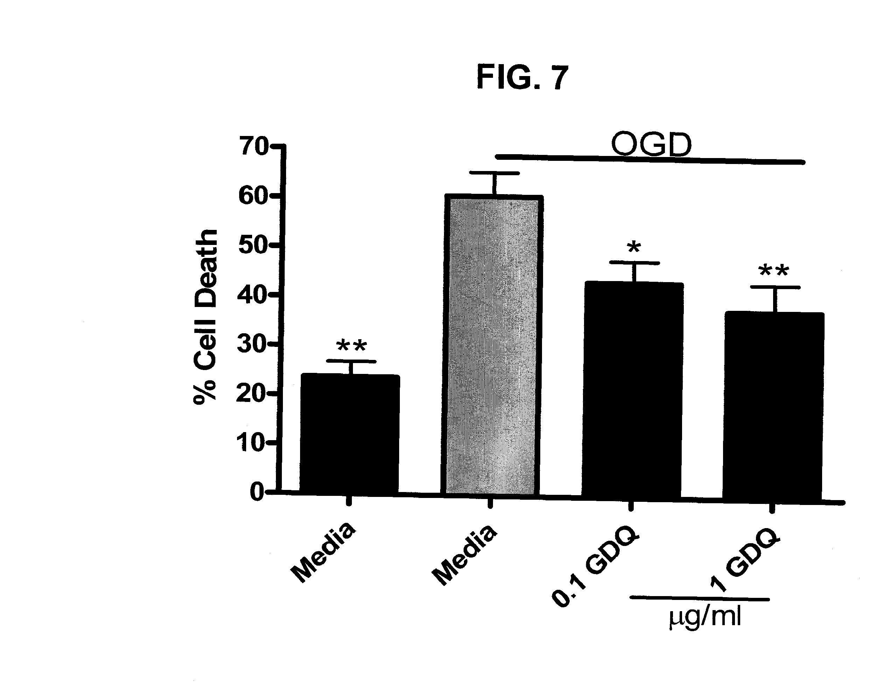

As shown in FIG. 7, Gardiquimod confers neuroprotection against oxygen-glucose deprivation in mouse neuronal cultures

### Example 6: Preconditioning with Gardiquimod in an in vivo Ischemic/Reperfusion Model

This example demonstrates that prophylactic administration of a composition containing Gardiquimod is neuroprotective in a mouse model of stroke.

Intraperitoneal Delivery: Preconditioning agent Gardiquimod in artificial cerebrospinal fluid (aCSF) or aCSF alone (control) was administered intraperitoneally to subject mice 72 hours prior to middle cerebral artery occlusion (MCAO) as described in Example 3.

**Motor Functions Tests:** Following pretreatment and/or ischemia/reperfusion, damage due to stroke was assessed using several behavioral indices of neurological function as described in Example 3.

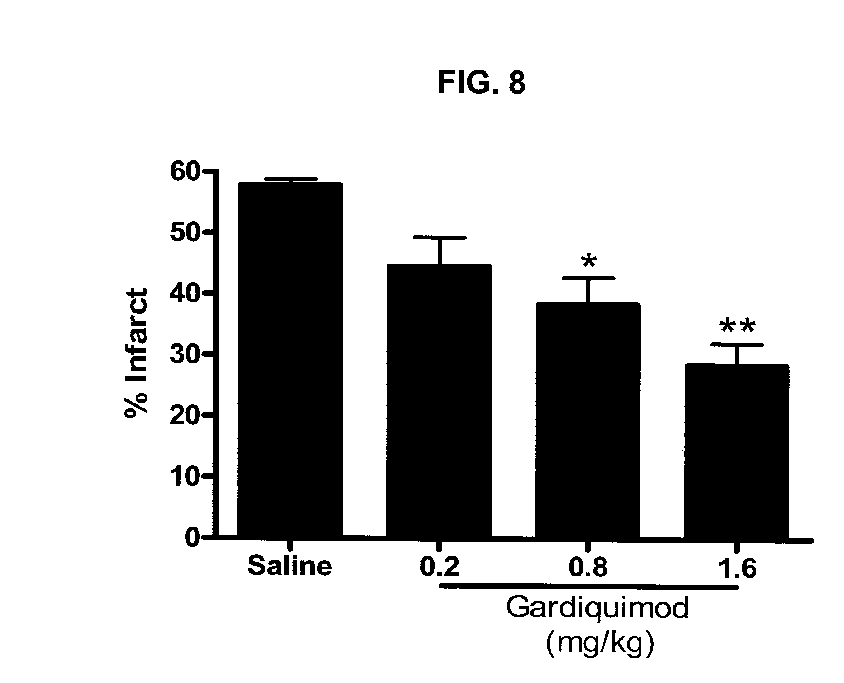

**Infarct calculations:** Performed as described in Example 3. As shown graphically in FIG. 8, percent infarct was significantly decreased in a dose-dependent fashion in mice treated with a preconditioning dose of Gardiquimod.

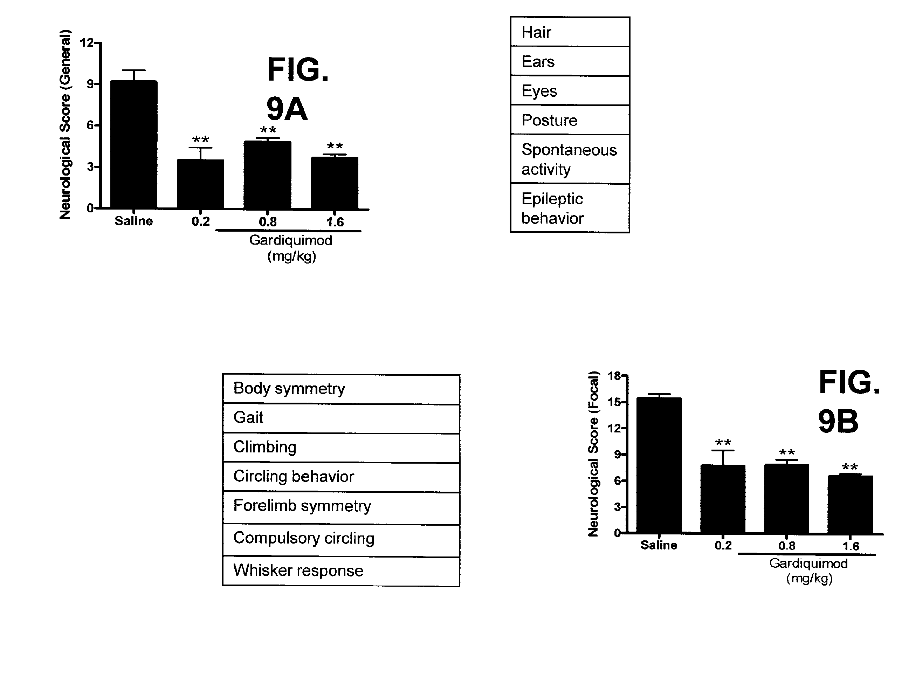

As shown graphically in FIGS. 9A-B a preconditioning dose of Gardiquimod also improved neurological deficit. All three doses of GDQ (subcutaneous; 0.2 - 1.6 mg/kg) reduced neurological deficits (both general and focal) 24 hours post MCAO.

### Example 7: Preconditioning with CL075 confers neuroprotection in an in vitro ischemia model

This example provides an exemplary *in vitro* model of neuronal ischemia, and demonstrates that preconditioning with CL075 (a TLR7/8 ligand) protects against hypoxia.

***In** vitro* mouse neuronal cultures: Cortical neuronal cultures were prepared as described in Example 1.

***In** vitro* neuronal ischemia model: Neuronal cultures were treated with 1 µg/ml CL075 in Neurobasal-A media supplemented with 1 % Glutamax 24 hours prior to 3 hours oxygen-glucose-deprivation (OGD) treatment. OGD was performed and cell analyzed as described in Example 1.

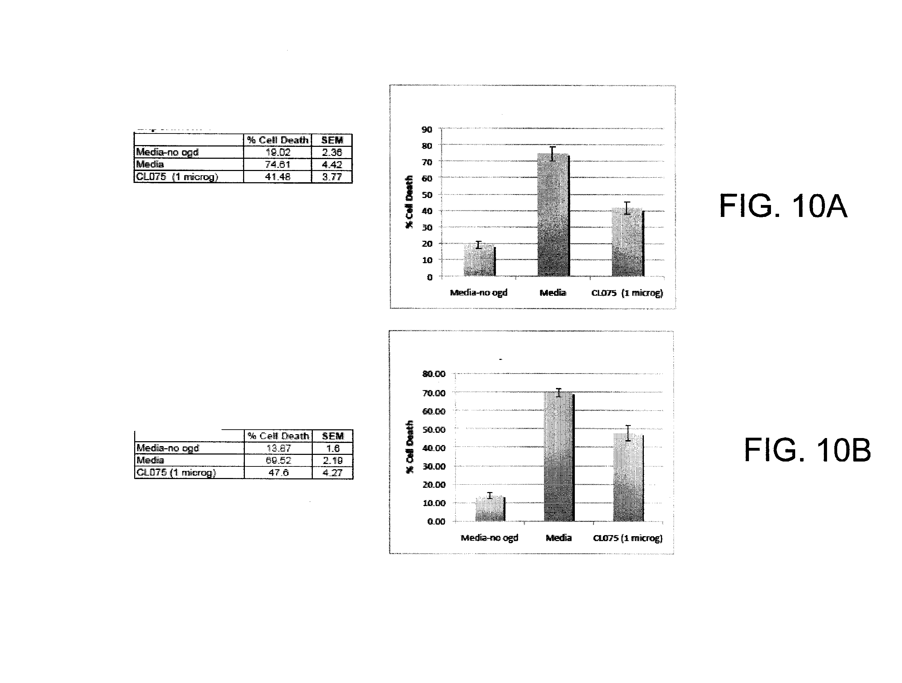

As shown in FIGS. 10A and 10B, CL075 confers neuroprotection against oxygen-glucose deprivation in mouse neuronal culture.

### Example 8: Preconditioning with Polyinosinic Polycytidylic Acid (Poly I:C) confers neuroprotection in an in vitro ischemia model

This example provides an exemplary *in vitro* model of neuronal ischemia, and demonstrates that preconditioning with Poly I:C (a TLR3 ligand) protects neural cells against ischemia.

***In vitro* mouse neuronal cultures:** Cortical neuronal cultures were prepared as described in Example 1.

***In vitro* neuronal ischemia model:** Neuronal cultures were treated with 1 µg/ml CL075 in Neurobasal-A media supplemented with 1 % Glutamax 24 hours prior to 3 hours oxygen-glucose-deprivation (OGD) treatment. OGD was performed and cell analyzed as described in Example 1.

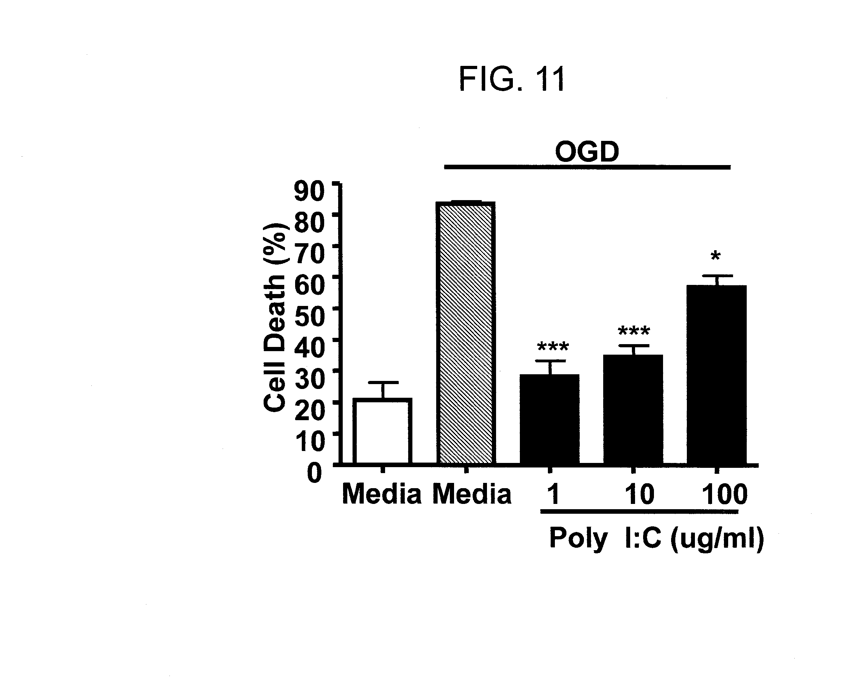

As shown in FIG. 11, Poly I:C treatment prior to oxygen-glucose deprivation induces neuroprotection against oxygen-glucose deprivation in mouse neuronal culture.

### Example 9: Preconditioning with Poly I:C in in vivo Ischemic/Reperfusion Models

This example demonstrates that prophylactic administration of a composition containing Poly I:C is neuroprotective and renal cell protective in a mouse model of stroke and renal ischemia, respectively.

Intraperitoneal Delivery: Preconditioning agent Poly I:C (25 µg) in artificial saline or saline alone (control) was administered intraperitoneally to subject mice 72 hours prior to middle cerebral artery occlusion (MCAO; 45 minutes) as described in Example 3 or 48 hours prior to bilateral renal clamping (45 min).

**Infarct calculations:** Performed as described in Example 3.

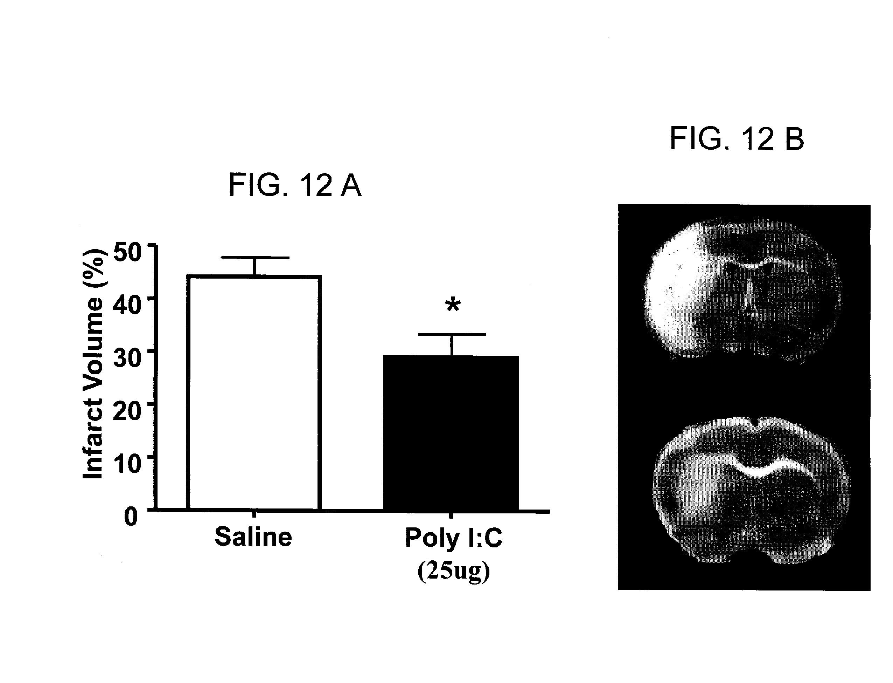

As shown in FIGS. 12A and 12B, percent infarct was significantly decreased in a dose-dependent fashion in mice treated with a preconditioning dose of Poly I:C. Poly I:C preconditioning significantly reduced brain damage at 72 hours following 45 minute middle cerebral artery occlusion.

**Creatinine levels:** To assess renal injury due to the ischemia, creatinine levels were measured 48 hours following reperfusion as follows. Mice were treated with saline or poly I:C (1.6mg/kg; sc) 48 hrs prior to renal ischemia. Just prior to surgery blood was collected via the saphenous vien for baseline determination of creatinine levels. Bilateral ischemia was induced as follows: mice were anesthestized with 3% isoflurane and maintained at 1.5-2% throughout surgery. A midline incision was made and both kidneys exteriorized and clamps placed on the artery and veins of each kidney for 45 minutes. After ischemia, the clamps were withdrawn inducing reperfusion and the incision sutured up. Mice were euthanized 48 hr post initiation of reperfusion and blood collected. Serum levels of creatinine were determined via a commercially available immunoassay.

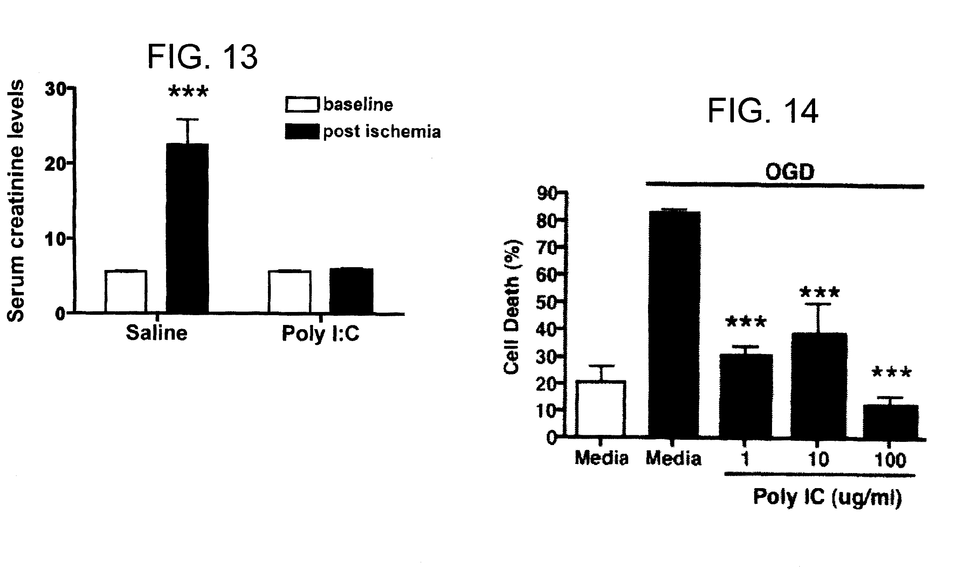

As shown in FIG. 13, serum levels of creatinine were dramatically increased in post ischemic mice following only saline injection. However, treatment with poly I:C prior to the renal ischemia reduced the injury to baseline levels.

### Example 10: Poly I:C Confers Neuroprotection in an in vitro Ischemia Model Following Injury

This example provides an exemplary *in vitro* model of neuronal ischemia, and demonstrates that administration of Poly I:C following oxygen-glucose deprivation protects neural cells against ischemia.

***In vitro* mouse neuronal cultures:** Cortical neuronal cultures were prepared as described in Example 1.

***In vitro* neuronal ischemia model:** Neuronal cultures were exposed to 3 hours oxygen-glucose-deprivation (OGD) treatment as described in Example 1. After OGD, cells were treated with 1 to 100 µg/ml Poly I:C. 24 hours later cells were analyzed as described in Example 1.

As shown in FIG. 14, Poly I:C treatment after oxygen-glucose deprivation induces neuroprotection in mouse neuronal culture. Based on these observations, the benefits of poly I:C treatment or other TLR3 ligand following ischemic events can be demonstrated *in vivo* using the brain and renal ischemia models described in Examples 3 and 9 (except that the TLR ligand would be administered following the event, instead of prior, for example at least 1 hour, at least 8 hours, at least 24 hours, or at least 48 hours later.

### Example 11 : TLR3 Knock-Out Mice Have Greater Damage from Ischemia in an in vivo Model of Stroke

This example demonstrates that TLR3 signaling provides a protective role *in vivo* in response to stroke.

Wild-type or TLR3 knock-out mice were subjected to 45 minute middle cerebral artery occlusion, and 24 hours following surgery the brains were stained with TTC and infarct volume was assessed as described n Example 3.

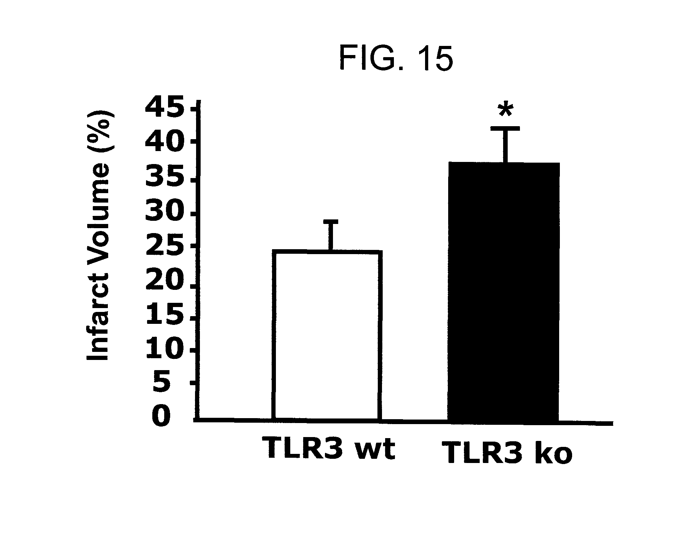

As shown in FIG. 15, the absence of functional TLR3 results in increased damage from ischemia as compared to mice with functional TLR3. TLR3 plays a protective role in the body's endogenous response to ischemia. mRNA is the endogenous ligand for TLR3. This protection may be due in part to IFNβ, a neuroprotectant induced by TLR3 stimulation.

### Example 12: Poly I:C Induces Production of TNF-a and IFN-β

This example demonstrates that Poly I:C increases both TNF-a and IFN-β in the serum of mice.

Mice were injected with saline, CpG (0.8 mg/kg) LPS (0.4 mg/kg) or Poly I:C (1 mg/kg) 2 hours prior to serum collection. Levels of TNF-a and IFN-β were measured using commercially available immunoassay kits.

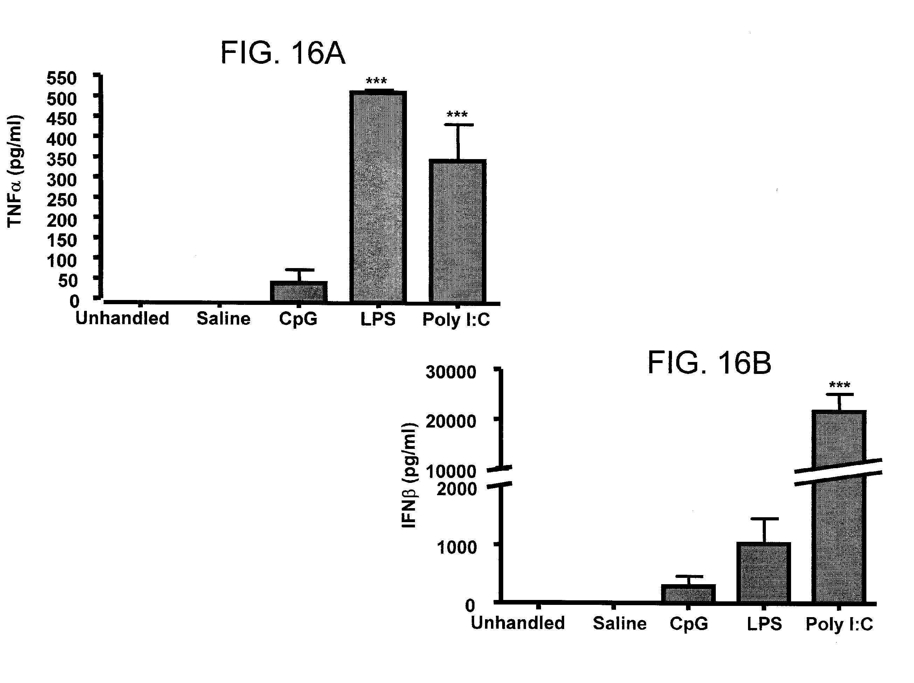

As shown in FIG. 16A, as with other TLR ligands, a small dose of Poly I:C increases TNFa levels, indicating similarities between TLR pathways. TNFa is required for CpG (TLR9) and LPS (TLR4) preconditioning. As shown in FIG. 16B, TLR3 stimulation with a protective dose of Poly I:C produces significantly more IFNβ than stimulation of TLR9 or TLR4.

### Example 13: Preconditioning with Resiquimod in vivo Reduces Ischemia Damage

This example demonstrates that resiquimod, as TLR7 ligand reduces damage in *an* in *vivo* model of cerebral ischemia.

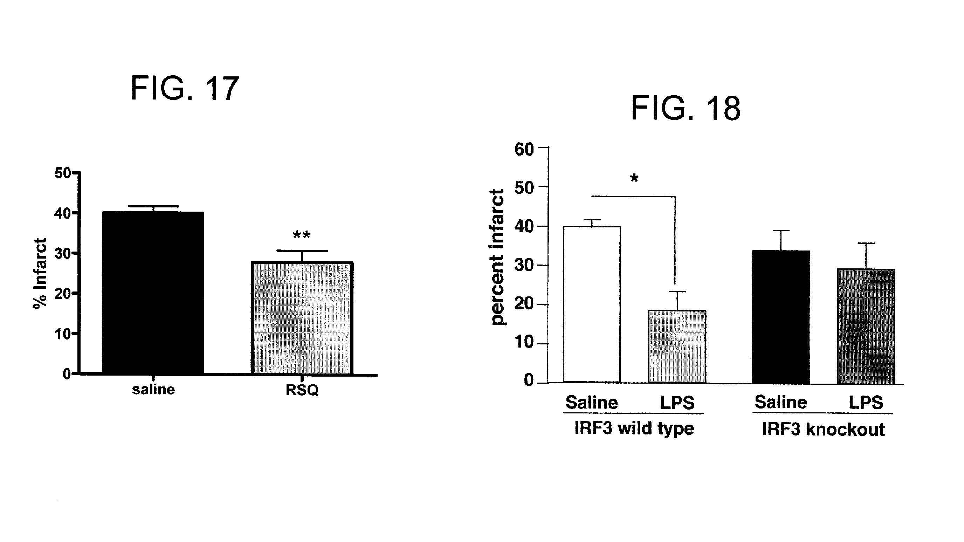

Mice were pre-treated with resiquimod (RSQ, 0.8 mg/kg, s.c.) 72 hours prior to MCAO, and infarct size was determined 24 hours following MCAO as described in Example 3. As shown in FIG. 17, pre-treating with RSQ provides neuroprotection against cerebral ischemia.

### Example 14: IRF3 is a Mediator of LPS Preconditioning

This example demonstrates that interferon regulatory factor-3 (IRF3) mediates the preconditioning response observed by LPS, a TLR4 ligand.

IRF3 knockout and wild-type mice were pre-treated with LPS (0.4 mg/kg) or saline 72 hours prior to MCAO (40 min), and infarct size was determined 24 hours following MCAO using TTC staining as described in Example 3. As shown in FIG. 18, in the absence of IRF3, no considerable neuroprotection is observed by pre-treating with LPS. Thus, IRF3 is needed to obtain neuroprotection with LPS preconditioning.

### Example 15: Preconditioning with a CpG oligonucleotide Mixture in an in vivo Ischemic/Reperfusion Model

This example demonstrates that prophylactic administration of a composition containing a mixture of CpG oligonucleotides, K-mix (SEQ ID NOS: 6-8), is neuroprotective in a primate model of stroke.

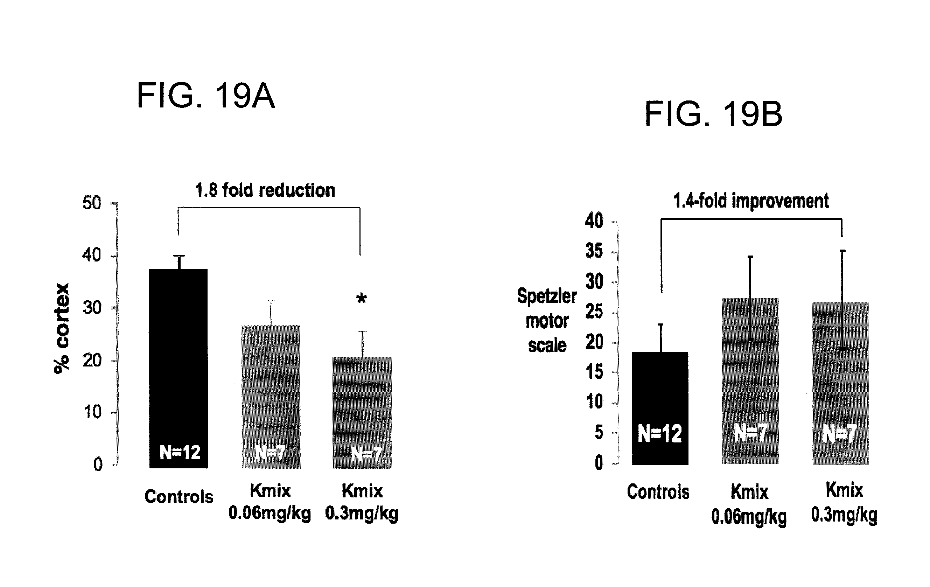

Male rhesus macaques were given an intramuscular injection of saline, or K-mix CpG ODN (0.06 mg/kg or 0.3 mg/kg) 3 days prior to 60 minute occlusion of the MCA and both ACAs. The protective effect was dose dependent and highly significant (p<0.005; **FIG.** 19A). Neurological assessment was also carried out on animals that underwent pre-treatment with CpG **(****FIG.** 19B) and these animals showed greater improvement of motor function at 2 days.

### Example 16: Other Ischemia Models

As shown in the examples above, TLR ligands have protective and treatment effects *in vitro* and *in vivo* for excitotoxic injury, ischemia, and hypoxia. One skilled in the art will appreciate that other TLR ligands or agonists can be tested for their ability to have desired protective or treatment effects on an excitotoxic injury, ischemia, or hypoxia.

For example, the effect of a TLR ligand (for example using the doses and modes of administration disclosed herein) on the ability to protect against ischemic injury, or treat a previous ischemic injury, can be determined using other *in vivo* ischemia models known in the art. To determine the effect of the TLR ligand on preconditioning, the ligand is administered prior to inducing ischemia as described above, while for determining the effect of the TLR ligand on treatment, the ligand is administered subsequent to inducing ischemia as described above.

For example, the protective or treatment effect of a TLR ligand on cardiac ischemia can be determined using the methods for inducing cardiac ischemia provided in Naderi et al. (Eur. J. Pharmacol. Phenylephrine produces late pharmacological preconditioning in the isolated rat heart. E-published 2009 Oct 30). Similarly, the protective or treatment effect of a TLR ligand on lung ischemia can be determined using the methods for inducing lung ischemia provided in Hirayama et al. (J. Heart Lung Transplant 28:1180-4, 2009). In addition, the protective or treatment effect of a TLR ligand on muscular ischemia can be determined using the methods for inducing hind limb ischemia provided in Shireman et al. (J. Surg. Res. 134:145-57, 2006) or Shireman (J. Vasc. Surg. 45:48A-56A, 2007). Additional methods for inducing ischemia are provided in Wenwu et al. (Pediatr. Cardiol. Limb Ischemic Preconditioning Reduces Heart and Lung Injury After an Open Heart Operation in Infants. E-published 29 Sept 2009).

### Example 17: Exemplary Model of TLR3 Neuroprotection

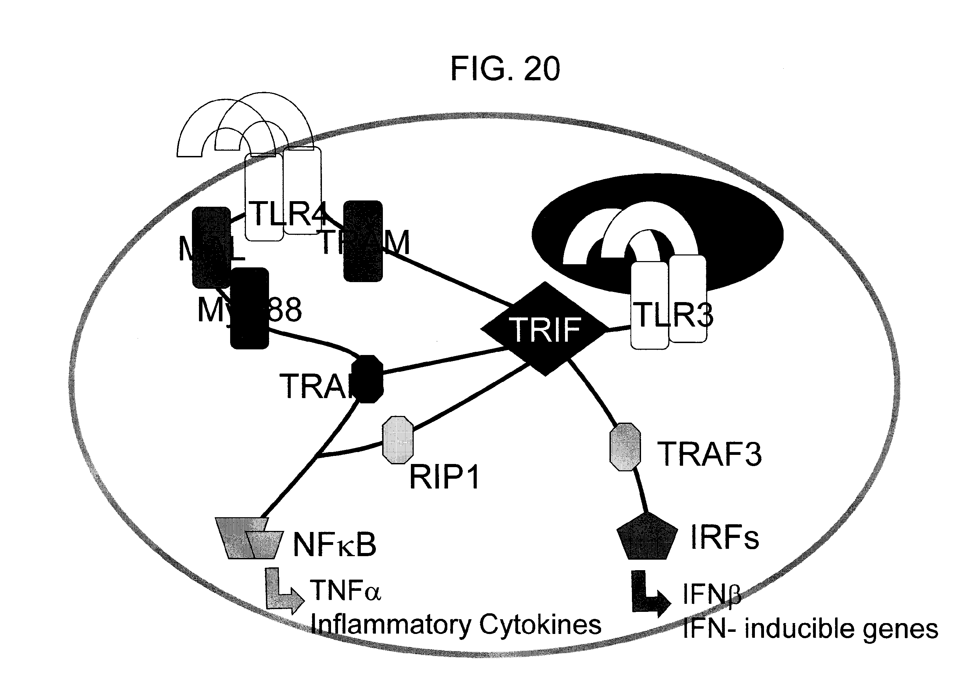

Without wishing to be bound to a particular theory, the model shown in FIG. 20 is proposed for TLR3 and TLR4 signalling. TLR3 signals through TRIF to activate the transcription factors NFκB and IRFs. TLR3 is the only TLR to signal solely through the adapter molecule TRIF which leads to robust production of IFNβ. The discovery that TLR3 is an endogenously protective pathway in ischemia and the discovery that preconditioning with the TLR3 agonist PolyI:C can protect the brain from ischemic damage demonstrates that TRIF signaling is neuroprotective. This observation is consistent with a protective role for interferon in ischemia and identifies mechanisms of TLR-mediated preconditioning.

In view of the many possible embodiments to which the principles of the disclosure may be applied, it should be recognized that the illustrated embodiments are only examples of the disclosure and should not be taken as limiting the scope of the invention. Rather, the scope of the disclosure is defined by the following claims. We therefore claim as our invention all that comes within the scope of these claims.

## Claims

1. A composition comprising a Toll-like receptor (TLR) ligand for use in a method of treating a cell in a subject having excitotoxic injury, ischemia, hypoxia, or combinations thereof comprising:
systemically administering to the subject the composition, thereby treating the cell for excitotoxic injury, ischemia, hypoxia, or combinations thereof.

2. A composition according to claim 1 for use in a method of protecting a cell in a subject against excitotoxic injury, ischemia, hypoxia, or combinations thereof comprising:
systemically administering to the subject the composition, thereby protecting the cell against excitotoxic injury, ischemia, hypoxia, or combinations thereof.

3. The composition of claim 1, wherein the method further comprises selecting a subject having previously suffered an excitotoxic event, ischemic event, hypoxic event, or combinations thereof.

4. The composition of claim 1 or 3, wherein the subject has suffered atrial fibrillation, one or more transient ischemic events, a stroke, hypertension, or combinations thereof.

5. The composition of any of the preceding claims, wherein the TLR ligand is a TLR1, TLR2, TLR3, TLR4, TLR5, TLR6, TLR7, TLR8, or TLR9 ligand; particularly, wherein the TLR2 ligand is MALP-2, the TLR3 ligand is poly I:C or dsRNA, the TLR4 ligand is a lipopolysaccharide (LPS), the TLR5 ligand is flagellin, the TLR6 ligand is a diacyl lipopeptide, the TLR7 ligand is Gardiquimod, CL075, imiquimod or resiquimod, the TLR8 ligand is CL075 or resiquimod, and the TLR9 ligand is a CpG oligonucleotide; or wherein the TLR ligand is not LPS; or, wherein the TLR ligand is a TLR3 ligand comprising poly I:C or a TLR7 ligand comprising Gardiquimod, CL075, imiquimod or resiquimod.

6. The composition of claims 1 or 3 to 5, wherein the method comprises administering the composition comprising the TLR ligand after an excitotoxic, ischemic or hypoxic event; or wherein the method comprises administering a plurality of doses of the composition comprising the TLR ligand.

7. The composition of any of the preceding claims, wherein the cell is a neural cell, a muscle cell, a liver cell, a kidney cell, an endothelial cell or an immune system cell.

8. The composition of claims 1 or 3 to 7, wherein the subject has suffered a stroke, epilepsy, traumatic brain injury, or Alzheimer's disease.

9. The composition of any of the preceding claims, wherein administering the composition results in increased production of a cytoprotective cytokine.

10. The composition of any of the preceding claims, wherein the TLR ligand is administered at a dose of at least 0.005 mg/kg and no more than 5 mg/kg.

11. The compositon of claim 2, wherein the method further comprises selecting a subject at risk for an excitotoxic event, ischemic event, hypoxic event, or combinations thereof.

12. The composition of claims 2 or 11, wherein the method comprises administering the composition comprising the TLR ligand prior to an excitotoxic, ischemic or hypoxic event.

13. A ligand that binds to and activates a TLR, which TLR is expressed by at least one cell of the central nervous system or the periphery, for use in a method of treating a neural cell in a subject having an excitotoxic injury, comprising:
systemically administering to the subject the ligand, thereby treating the neural cell in the subject having the excitotoxic injury; particularly, further comprising selecting a subject who has suffered a previous excitotoxic event.

14. A ligand that binds to a TLR expressed by at least one cell of a tissue other than the central nervous system for use in a method of treating a non-neural cell in a subject having suffered ischemia, comprising:
systemically administering to a subject the ligand, thereby treating the non-neural cell.

15. The method of claim 14, wherein the non-neural cell is a kidney cell; or, wherein the ischemia is associated with a surgical procedure.
